(19) Europäisches Patentamt
European Patent Office
Office européen des brevets

(11) EP 2 883 959 A1

(12) # EUROPEAN PATENT APPLICATION

(43) Date of publication:
**17.06.2015 Bulletin 2015/25**

(51) Int Cl.:
***C12P 19/30*** *(2006.01)*

(21) Application number: **13197287.9**

(22) Date of filing: **13.12.2013**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Plasmia Biotech, S.L.**
**08911 Barcelona (ES)**

(72) Inventors:
- **Pascual Gilabert, Marta**
**08100 Barcelona (ES)**
- **Deroncelé Thomas, Víctor M.**
**08100 Barcelona (ES)**
- **Montilla Arévalo, Rafael**
**08100 Barcelona (ES)**

(74) Representative: **Illescas, Manuel**
**Manuel Illescas y Asocioados, S.L. (MIA)**
**Principe de Vergara n° 197**
**Oficina 1°A**
**28002 Madrid (ES)**

(54) **Enzymatic production of cytosinic nucleoside analogues**

(57) The invention relates to a new synthesis process of cytosine nucleoside analogues by using nucleoside phosphorylase enzymes, particularly Pyrimidin Nucleoside Phosphorylases (PyNPs) or mixtures of Purine Nucleoside Phosphorylases (PNPs) and PyNPs.

EP 2 883 959 A1

SEQUENCE LISTING

<110> Plasmia Biotech

<120> Enzymatic production of cytosinic nucleoside analogues

<130> EP-06550

<160> 12

<170> BiSSAP 1.2

<210> 1
<211> 825
<212> DNA
<213> Sulfolobus solfataricus

<220>
<221> source
<222> 1..825
<223> /mol_type="unassigned DNA"
/note="P2 Purine nucleoside phosporylase (deoD)"
/organism="Sulfolobus solfataricus"

<400> 1
```
gtgccatttt tagaaaatgg ttccatggta tatggtgatt tcattagaaa tcaagaggta      60
agaaaaagaa ttacaaagga agaacttggg atagaagaag acgaaatccc ggaaagggta     120
gttgtaacac ctatgccatt taatactcaa tttcctaaaa actttgaaga tactttaact     180
aacttaggaa ttaaagtaaa taggttaaaa gtggaagacc aaatacttag acaattcgga     240
ggaaatttat tgcttgaaaa agacggtaat agaggattta ttgcgttcat aggcagaggt     300
ctgatagatt tcactgagag gataaggatt ttagctacag tttcgcgcat taaagatata     360
ttatttattg gtactgcagg atcgttatct aatgaaatat taataggaga tctaaatata     420
ccaaaatacg ccatcccatt cgaaaacgta agtgattttt acgctgatcc taccatagca     480
attccacaag ctgatgaaaa gttgctgaac gaagtttatg agtacgctga ggaaactgga     540
gttaaaaccc actcaacctt acatgcaaca ctacttttcc cttattccga aactactgag     600
ttcctaaact acttattaaa tatcggcgtt tctacgatag atatggaagt cagtgctttt     660
tataagatgt ctagatttta cggtaaaaga gctgttgcag tattacgaat ttcagatatg     720
cctttaatag aactgcataa gcaagaggaa ttgattaagg caagaaggga aattgcagtt     780
aatgctgttt tcagaattac cttaagattc ttaaaactga tttaa                     825
```

<210> 2
<211> 849
<212> DNA
<213> Aeropyrum pernix K1

<220>
<221> source
<222> 1..849
<223> /mol_type="unassigned DNA"

```
        /note="udp uridine phosphorylase"
        /organism="Aeropyrum pernix K1"

<400> 2
ttgggagacg agagtctaag gagcgccgcc cgtcccgagg gggagggagg gctgcagtac      60

catctgaggg tcaggagggg ggatgtggcc cgctacgttc tcctcccggg agaccccgag     120

aggacagacc ttatagcccg cctctgggat gaagcgaggc ttgtagcgca ccaccgggag     180

tacaggacgt ggaccggctt ctacaagggg acatcgataa gtgtaacaag caccgggata     240

ggctctccca gcacggcgat agccgttgag gagctgctga gggttggagc cgagactttc     300

ataagagtag gcactatggg cggtataagg gaggatctgc ggcccggcac cctggttata     360

gggagtgcgg cggttaggat ggaggggacg agcggccagt acgctccccg ggggttccca     420

gcggccgcca gctatgacgt tgtggcggcg ctggtggagg ctgctgaggc gctcggggtt     480

aggtatgagg ttggcgttgt tgccagcacg gacagcttct acctgggcca ggggaggccg     540

gggtacgggg ggtatatgac gccggaggct tcggaagtca tacccctcct caggtcagcc     600

ggcgtcctcg gcttcgagat ggaggcctcc gccctcttca ccctatccca gctctacggc     660

gccagggcag ggtgcgtgtg cgcggtagtg gcaaacaggg ttagcgggga gtttgtggta     720

aacgcggggg ttgaagacgc tgctagggtt gcctccgagg cggtagccat actagcaggc     780

tgggacaggg agagggagaa gaggggtaag aaatggtttt acccgagcct ggcgtgcaga     840

cgcacatag                                                             849


<210> 3
<211> 274
<212> PRT
<213> Sulfolobus solfataricus P2

<220>
<223> Purine nucleoside phosporylase (DeoD)

<400> 3
Met Pro Phe Leu Glu Asn Gly Ser Met Val Tyr Gly Asp Phe Ile Arg
1               5                   10                  15
Asn Gln Glu Val Arg Lys Arg Ile Thr Lys Glu Glu Leu Gly Ile Glu
            20                  25                  30
Glu Asp Glu Ile Pro Glu Arg Val Val Val Thr Pro Met Pro Phe Asn
        35                  40                  45
Thr Gln Phe Pro Lys Asn Phe Glu Asp Thr Leu Thr Asn Leu Gly Ile
    50                  55                  60
Lys Val Asn Arg Leu Lys Val Glu Asp Gln Ile Leu Arg Gln Phe Gly
65                  70                  75                  80
Gly Asn Leu Leu Leu Glu Lys Asp Gly Asn Arg Gly Phe Ile Ala Phe
                85                  90                  95
Ile Gly Arg Gly Leu Ile Asp Phe Thr Glu Arg Ile Arg Ile Leu Ala
            100                 105                 110
Thr Val Ser Arg Ile Lys Asp Ile Leu Phe Ile Gly Thr Ala Gly Ser
        115                 120                 125
Leu Ser Asn Glu Ile Leu Ile Gly Asp Leu Asn Ile Pro Lys Tyr Ala
    130                 135                 140
Ile Pro Phe Glu Asn Val Ser Asp Phe Tyr Ala Asp Pro Thr Ile Ala
```

```
145                 150                 155                 160
Ile Pro Gln Ala Asp Glu Lys Leu Leu Asn Glu Val Tyr Glu Tyr Ala
                165                 170                 175
Glu Glu Thr Gly Val Lys Thr His Ser Thr Leu His Ala Thr Leu Leu
            180                 185                 190
Phe Pro Tyr Ser Glu Thr Thr Glu Phe Leu Asn Tyr Leu Leu Asn Ile
        195                 200                 205
Gly Val Ser Thr Ile Asp Met Glu Val Ser Ala Phe Tyr Lys Met Ser
    210                 215                 220
Arg Phe Tyr Gly Lys Arg Ala Val Ala Val Leu Arg Ile Ser Asp Met
225                 230                 235                 240
Pro Leu Ile Glu Leu His Lys Gln Glu Glu Leu Ile Lys Ala Arg Arg
                245                 250                 255
Glu Ile Ala Val Asn Ala Val Phe Arg Ile Thr Leu Arg Phe Leu Lys
            260                 265                 270
Leu Ile


<210> 4
<211> 282
<212> PRT
<213> Aeropyrum pernix K1

<220>
<223> Uridine phosphorylase

<400> 4
Met Gly Asp Glu Ser Leu Arg Ser Ala Ala Arg Pro Glu Gly Glu Gly
1               5                   10                  15
Gly Leu Gln Tyr His Leu Arg Val Arg Arg Gly Asp Val Ala Arg Tyr
            20                  25                  30
Val Leu Leu Pro Gly Asp Pro Glu Arg Thr Asp Leu Ile Ala Arg Leu
        35                  40                  45
Trp Asp Glu Ala Arg Leu Val Ala His His Arg Glu Tyr Arg Thr Trp
    50                  55                  60
Thr Gly Phe Tyr Lys Gly Thr Ser Ile Ser Val Thr Ser Thr Gly Ile
65                  70                  75                  80
Gly Ser Pro Ser Thr Ala Ile Ala Val Glu Glu Leu Leu Arg Val Gly
                85                  90                  95
Ala Glu Thr Phe Ile Arg Val Gly Thr Met Gly Gly Ile Arg Glu Asp
            100                 105                 110
Leu Arg Pro Gly Thr Leu Val Ile Gly Ser Ala Ala Val Arg Met Glu
        115                 120                 125
Gly Thr Ser Gly Gln Tyr Ala Pro Arg Gly Phe Pro Ala Ala Ala Ser
    130                 135                 140
Tyr Asp Val Val Ala Ala Leu Val Glu Ala Ala Glu Ala Leu Gly Val
145                 150                 155                 160
Arg Tyr Glu Val Gly Val Val Ala Ser Thr Asp Ser Phe Tyr Leu Gly
                165                 170                 175
Gln Gly Arg Pro Gly Tyr Gly Gly Tyr Met Thr Pro Glu Ala Ser Glu
            180                 185                 190
Val Ile Pro Leu Leu Arg Ser Ala Gly Val Leu Gly Phe Glu Met Glu
        195                 200                 205
Ala Ser Ala Leu Phe Thr Leu Ser Gln Leu Tyr Gly Ala Arg Ala Gly
    210                 215                 220
Cys Val Cys Ala Val Val Ala Asn Arg Val Ser Gly Glu Phe Val Val
225                 230                 235                 240
Asn Ala Gly Val Glu Asp Ala Ala Arg Val Ala Ser Glu Ala Val Ala
                245                 250                 255
Ile Leu Ala Gly Trp Asp Arg Glu Arg Glu Lys Arg Gly Lys Lys Trp
            260                 265                 270
Phe Tyr Pro Ser Leu Ala Cys Arg Arg Thr
        275                 280
```

<210> 5
<211> 720
<212> DNA
<213> Thermofilum pendens Hrk 5

<220>
<221> source
<222> 1..720
<223> /mol_type="unassigned DNA"
/note="purine-nucleoside phosphorylase"
/organism="Thermofilum pendens Hrk 5"

<400> 5
```
gtggctaagc cgttacacat actcgcaaag ccggaggaca tagcccccag ggttatcgcc      60

tcgggggacc ccgccagagt gaagcaactc tcaagctacc tcgataaccc caggctggtg     120

aacgagaaca ggggcttcct ggtatacacc ggcacgtaca agggggtacc cgtgactgtt     180

gctacccaca tgataggtgc tccctccgcc gcgatagtct tcgaggagct cataatgctg     240

ggtgcgaagc tgatagtcag gttcggcacc tgcggcggct tcctgccgga gatgcgcgta     300

ggggacttcg tgatagcgac gggcgcctcg tacagcggtg gggaacaat gaatacctac      360

agccccggcg agtgcatggc cgccgtgccg gactacgacg tgctcagcgc actcgtcgag     420

agcgcttcga ggcacgggtt gaagtacttc ctggggcccg tggttagcag cgataacttc     480

tactcaggta tagagtacct gaacaggtgg ataaacaggg gcatgatagc cgtcgacatg     540

gaggctgcca cgctgttcgt cgtcggcagg cttaggcgcg tgaagaccgg tgcctccttc     600

gtcgtgagcg acgtgatcgg tgaggcttac aagaagatgg cgacggccga ggagctacgc     660

gaggctgtcg acaaggcttc gagagccgtg ctagacgcgg ttataagcgt gaaagtttga     720
```

<210> 6
<211> 239
<212> PRT
<213> Thermofilum pendens Hrk 5

<220>
<223> Purine-nucleoside phosphorylase

<400> 6
```
Met Ala Lys Pro Leu His Ile Leu Ala Lys Pro Glu Asp Ile Ala Pro
1               5                   10                  15
Arg Val Ile Ala Ser Gly Asp Pro Ala Arg Val Lys Gln Leu Ser Ser
            20                  25                  30
Tyr Leu Asp Asn Pro Arg Leu Val Asn Glu Asn Arg Gly Phe Leu Val
        35                  40                  45
Tyr Thr Gly Thr Tyr Lys Gly Val Pro Val Thr Val Ala Thr His Met
    50                  55                  60
Ile Gly Ala Pro Ser Ala Ala Ile Val Phe Glu Glu Leu Ile Met Leu
65                  70                  75                  80
Gly Ala Lys Leu Ile Val Arg Phe Gly Thr Cys Gly Gly Phe Leu Pro
                85                  90                  95
Glu Met Arg Val Gly Asp Phe Val Ile Ala Thr Gly Ala Ser Tyr Ser
            100                 105                 110
Gly Gly Gly Thr Met Asn Thr Tyr Ser Pro Gly Glu Cys Met Ala Ala
```

```
        115                 120                 125
Val Pro Asp Tyr Asp Val Leu Ser Ala Leu Val Glu Ser Ala Ser Arg
    130                 135                 140
His Gly Leu Lys Tyr Phe Leu Gly Pro Val Val Ser Ser Asp Asn Phe
145                 150                 155                 160
Tyr Ser Gly Ile Glu Tyr Leu Asn Arg Trp Ile Asn Arg Gly Met Ile
                165                 170                 175
Ala Val Asp Met Glu Ala Ala Thr Leu Phe Val Val Gly Arg Leu Arg
            180                 185                 190
Arg Val Lys Thr Gly Ala Ser Phe Val Val Ser Asp Val Ile Gly Glu
        195                 200                 205
Ala Tyr Lys Lys Met Ala Thr Ala Glu Glu Leu Arg Glu Ala Val Asp
    210                 215                 220
Lys Ala Ser Arg Ala Val Leu Asp Ala Val Ile Ser Val Lys Val
225                 230                 235

<210> 7
<211> 717
<212> DNA
<213> Staphylothermus marinus F1

<220>
<221> source
<222> 1..717
<223> /mol_type="unassigned DNA"
      /note="Purine-nucleoside phosphorylase "
      /organism="Staphylothermus marinus F1"

<400> 7
atgaagcctg ctcttctaaa agatgttgca ggggttagtg atctagttat tgtaatgggt      60

gatcctgata gagtatactt attatcaaca ttgctggaaa accccaagat catatatgat     120

aggagaggaa tagttgttgt taatggagaa tataagggta gaaaaataac acttgccagc     180

cacggtattg gttgtccaat ggcatcaatt atattggagg aactgggtat gcttggtgct     240

aaaacaatta ttagaatagg cacagctggc tcacttgttg aaaacattgg tttaggcgat     300

attgtgttag tagcgggggc tgggtacatg cttaatgggt gtggaaacaa tatgtattct     360

cccgaaataa atggaggaac aagcccagat cctctactgc tcagcgaaat ctatcattat     420

ttatcacatt ataatattaa gccacatatt ggactagtat ttacaagcga tgcattctat     480

gcggaagaaa atataattga taaactaacc aataaaggat tcatcgctgt tgatatggag     540

acagcaattc tatacatgct tggatggatg aggaagtgga ggacactatc aatactggta     600

gtaagcaaca gcctcgtgaa gaaaacaccg ttgcttacaa catatgagct cgcagagaag     660

tttgttgaac tagcaaaact agtattagaa tatcttgcaa gaaacactca gcactaa        717


<210> 8
<211> 238
<212> PRT
<213> Staphylothermus marinus F1

<220>
<223> Purine-nucleoside phosphorylase

<400> 8
```

```
Met Lys Pro Ala Leu Leu Lys Asp Val Ala Gly Val Ser Asp Leu Val
1               5                   10                  15
Ile Val Met Gly Asp Pro Asp Arg Val Tyr Leu Leu Ser Thr Leu Leu
            20                  25                  30
Glu Asn Pro Lys Ile Ile Tyr Asp Arg Arg Gly Ile Val Val Val Asn
        35                  40                  45
Gly Glu Tyr Lys Gly Arg Lys Ile Thr Leu Ala Ser His Gly Ile Gly
    50                  55                  60
Cys Pro Met Ala Ser Ile Ile Leu Glu Glu Leu Gly Met Leu Gly Ala
65                  70                  75                  80
Lys Thr Ile Ile Arg Ile Gly Thr Ala Gly Ser Leu Val Glu Asn Ile
                85                  90                  95
Gly Leu Gly Asp Ile Val Leu Val Ala Gly Ala Gly Tyr Met Leu Asn
            100                 105                 110
Gly Cys Gly Asn Asn Met Tyr Ser Pro Glu Ile Asn Gly Gly Thr Ser
        115                 120                 125
Pro Asp Pro Leu Leu Leu Ser Glu Ile Tyr His Tyr Leu Ser His Tyr
    130                 135                 140
Asn Ile Lys Pro His Ile Gly Leu Val Phe Thr Ser Asp Ala Phe Tyr
145                 150                 155                 160
Ala Glu Glu Asn Ile Ile Asp Lys Leu Thr Asn Lys Gly Phe Ile Ala
                165                 170                 175
Val Asp Met Glu Thr Ala Ile Leu Tyr Met Leu Gly Trp Met Arg Lys
            180                 185                 190
Trp Arg Thr Leu Ser Ile Leu Val Val Ser Asn Ser Leu Val Lys Lys
        195                 200                 205
Thr Pro Leu Leu Thr Thr Tyr Glu Leu Ala Glu Lys Phe Val Glu Leu
    210                 215                 220
Ala Lys Leu Val Leu Glu Tyr Leu Ala Arg Asn Thr Gln His
225                 230                 235
```

<210> 9
<211> 843
<212> DNA
<213> Hyperthermus butylicus DSM 5456

<220>
<221> source
<222> 1..843
<223> /mol_type="unassigned DNA"
/note="Uridine phosphorylase"
/organism="Hyperthermus butylicus DSM 5456"

<400> 9

```
gtggagaggc cctcggcgaa agcgccaacc gttgagggta aaatgtacca tatcatgctc      60

ggcccaggcg aaattccgcc ctacgtcctc ctgccaggcg atccaggtag gatagacgat     120

atagtcgcga cgtgggatga atggcgggag ctagcatttc accgcgagta tcgtagtgtc     180

aagggcaggt ataagggtgt ggaaataggc gctgtcagca ctggcatagg cggaccgtca     240

actgcaattg ccgtcgagga gctggctagg atagggggtac atacgttcat ccgcgtgggc     300

accactggcg ctatacaacc ggatatagaa cttggaacag tcatcatagg ctatgcagct     360

gtaagatatg atggtgctag tggtgagtat gctccgccag agtaccccgc agctgcgaca     420

cccgaggtgg ttctagctct tgttgaggct gctgagaggc tcggggtacc ataccgtgtc     480

ggcgtggtgg cctcaacagc aacattccac ttggggcaga gccgcccagg gttccgtggg     540

tacgagtgga gccgtagcag ggagaggcta gcagacctac agcgcatggg tgtcctcagc     600
```

ttcgagatgg aggcggcgac gatattcact ctcgcgagtc tctatgggct gcgggcaggc 660

tgtgtctgcg ccgcaatagc caaccgcgtg accgacgagt ttaaaccggg ggtcggggtt 720

agggaggcga tactagtggc taatgaggct gtgaggatac tagccgaggc cgacagggaa 780

aagggcagga agccagctag tataacaacg ctctacaacg ccgtcagaaa gctgtacggc 840

tag 843

<210> 10
<211> 280
<212> PRT
<213> Hyperthermus butylicus DSM 5456

<220>
<223> Uridine phosphorylase

<400> 10
Met Glu Arg Pro Ser Ala Lys Ala Pro Thr Val Glu Gly Lys Met Tyr
1 5 10 15
His Ile Met Leu Gly Pro Gly Glu Ile Pro Pro Tyr Val Leu Leu Pro
20 25 30
Gly Asp Pro Gly Arg Ile Asp Asp Ile Val Ala Thr Trp Asp Glu Trp
35 40 45
Arg Glu Leu Ala Phe His Arg Glu Tyr Arg Ser Val Lys Gly Arg Tyr
50 55 60
Lys Gly Val Glu Ile Gly Ala Val Ser Thr Gly Ile Gly Gly Pro Ser
65 70 75 80
Thr Ala Ile Ala Val Glu Glu Leu Ala Arg Ile Gly Val His Thr Phe
85 90 95
Ile Arg Val Gly Thr Thr Gly Ala Ile Gln Pro Asp Ile Glu Leu Gly
100 105 110
Thr Val Ile Ile Gly Tyr Ala Ala Val Arg Tyr Asp Gly Ala Ser Gly
115 120 125
Glu Tyr Ala Pro Pro Glu Tyr Pro Ala Ala Ala Thr Pro Glu Val Val
130 135 140
Leu Ala Leu Val Glu Ala Ala Glu Arg Leu Gly Val Pro Tyr Arg Val
145 150 155 160
Gly Val Val Ala Ser Thr Ala Thr Phe His Leu Gly Gln Ser Arg Pro
165 170 175
Gly Phe Arg Gly Tyr Glu Trp Ser Arg Ser Arg Glu Arg Leu Ala Asp
180 185 190
Leu Gln Arg Met Gly Val Leu Ser Phe Glu Met Glu Ala Ala Thr Ile
195 200 205
Phe Thr Leu Ala Ser Leu Tyr Gly Leu Arg Ala Gly Cys Val Cys Ala
210 215 220
Ala Ile Ala Asn Arg Val Thr Asp Glu Phe Lys Pro Gly Val Gly Val
225 230 235 240
Arg Glu Ala Ile Leu Val Ala Asn Glu Ala Val Arg Ile Leu Ala Glu
245 250 255
Ala Asp Arg Glu Lys Gly Arg Lys Pro Ala Ser Ile Thr Thr Leu Tyr
260 265 270
Asn Ala Val Arg Lys Leu Tyr Gly
275 280

<210> 11
<211> 846
<212> DNA
<213> Acidilobus saccharovorans 345-15

<220>
<221> source
<222> 1..846
<223> /mol_type="unassigned DNA"
/note="Uridine phosphorylase"
/organism="Acidilobus saccharovorans 345-15"

<400> 11
gtggaggagc gcatgtcttc agcttcaagg cccttcgact cggagggcag ggcctaccac 60

ctgggcctca ggaagggcga cgtgccaaag tacgtgctta tgccaggcga ggttgagagg 120

gcctcaagga tagcctcctc ctgggacagg agctggaggc tggcccagag gagggagtac 180

tcaagcttca ggggcgttta caggggcgtt gacgtggcgg tggtctccac gggcataggg 240

ggacctgcca ccgccatagc agttgaggag ctgctggagc tcggcgctga cacgctgata 300

agggttggaa gcaccggcgc catacaggat gacatagaag ttggggatat aataataacc 360

accgccgcgg tcaggatgga cggcacgagc taccagtacg ccccggccgg ctacccggcc 420

tcggccagct acgaggtaat catggccctg gtcgaggccg ccgagagcct cggggtgagg 480

taccaccttg gcatcacggc ctctactgac agcttctacg tgggccaggg gaggccgggc 540

tacgggggtt acatgccgag ctggtcaagg aacctcgtgc ctgacctgag gcagatgagg 600

gtgctgaact ttgagatgga gagcgccacc ctccttacct tagcaaatat atacggcttc 660

agggcggggg cagtgcacgc cgtctacgcc cagagggtta aggatgagtt cgttgcccac 720

gctggcgagg agaacctgat aaaggtggct gacgaggccg tgaggatact tcacgagtgg 780

gacgaggtta aggggagggc cgggaagagg tacttctacc cctcgctcct gaggcctggc 840

ccctga 846

<210> 12
<211> 281
<212> PRT
<213> Acidilobus saccharovorans 345-15

<220>
<223> Uridine phosphorylase

<400> 12
Met Glu Glu Arg Met Ser Ser Ala Ser Arg Pro Phe Asp Ser Glu Gly
1               5                   10                  15
Arg Ala Tyr His Leu Gly Leu Arg Lys Gly Asp Val Pro Lys Tyr Val
            20                  25                  30
Leu Met Pro Gly Glu Val Glu Arg Ala Ser Arg Ile Ala Ser Ser Trp
        35                  40                  45
Asp Arg Ser Trp Arg Leu Ala Gln Arg Arg Glu Tyr Ser Ser Phe Arg
    50                  55                  60
Gly Val Tyr Arg Gly Val Asp Val Ala Val Val Ser Thr Gly Ile Gly
65                  70                  75                  80
Gly Pro Ala Thr Ala Ile Ala Val Glu Glu Leu Leu Glu Leu Gly Ala
                85                  90                  95
Asp Thr Leu Ile Arg Val Gly Ser Thr Gly Ala Ile Gln Asp Asp Ile
            100                 105                 110
Glu Val Gly Asp Ile Ile Ile Thr Thr Ala Ala Val Arg Met Asp Gly

```
            115                     120                     125
Thr Ser Tyr Gln Tyr Ala Pro Ala Gly Tyr Pro Ala Ser Ala Ser Tyr
        130                     135                     140
Glu Val Ile Met Ala Leu Val Glu Ala Ala Glu Ser Leu Gly Val Arg
145                     150                     155                     160
Tyr His Leu Gly Ile Thr Ala Ser Thr Asp Ser Phe Tyr Val Gly Gln
                165                     170                     175
Gly Arg Pro Gly Tyr Gly Gly Tyr Met Pro Ser Trp Ser Arg Asn Leu
            180                     185                     190
Val Pro Asp Leu Arg Gln Met Arg Val Leu Asn Phe Glu Met Glu Ser
        195                     200                     205
Ala Thr Leu Leu Thr Leu Ala Asn Ile Tyr Gly Phe Arg Ala Gly Ala
    210                     215                     220
Val His Ala Val Tyr Ala Gln Arg Val Lys Asp Glu Phe Val Ala His
225                     230                     235                     240
Ala Gly Glu Glu Asn Leu Ile Lys Val Ala Asp Glu Ala Val Arg Ile
                245                     250                     255
Leu His Glu Trp Asp Glu Val Lys Gly Arg Ala Gly Lys Arg Tyr Phe
            260                     265                     270
Tyr Pro Ser Leu Leu Arg Pro Gly Pro
        275                     280
```

## Description

### Field of the invention

The present invention relates to a novel enzymatic process for the production of cytosine Nucleoside Analogues (NAs), in particular for the industrial production of cytosinic NAs active as pharmaceutically relevant antiviral and anticancer drugs, intermediates or prodrugs thereof.

### Background of the invention

Nucleoside analogues (NAs) are synthetic compounds structurally related to natural nucleoside. In terms of their structure, nucleosides are constituted by three key elements: (i) the hydroxymethyl group, (ii) the heterocyclic nitrogenous base moiety, and (iii) the furanose ring, which in several instances seems to act as a spacer presenting the hydroxymethyl group and the base in the correct orientation.

NAs are characterized by great structural diversity, since they exhibit variously modified carbohydrate and/or aglycon fragments.

NAs are extensively used as antiviral and antitumor agents. These molecules have been traditionally synthesized by different chemical methods which often require time-consuming multistep processes including protection-deprotection reactions on the heterocycle base and/or the pentose moiety to allow the modification of naturally occurring nucleosides (Boryski J. 2008. Reactions of transglycosylation in the nucleoside chemistry. Curr Org Chem 12:309-325). This time consuming multistep processes often lead to low yields and increased costs. Indeed, chemical methods usually increase the difficulty of obtaining products with correct stereo- and regioselectivity, generating by-products as impurities (Condezo, L. A., et al. 2007. Enzymatic synthesis of modified nucleosides, p. 401-423. Biocatalysis in the pharmaceutical and biotechnology industries. CRC Press, Boca Raton, FL, Mikhailopulo, I. A. 2007; Sinisterra, J.V. et al. 2010. Enzyme-catalyzed synthesis of nonnatural or modified nucleosides, p. 1-25. Encyclopedia of Industrial Biotechnology: Bioprocess, Bioseparation, and Cell Technology, John Wiley & sons, Ed. By M. C. Flickinger, 2010). Moreover, the chemical methods include the use of chemical reagents and organic solvents that are expensive and environmentally harmful.

Enzymatic synthesis of nucleosides involves the use of enzymes that catalyze the condensation of heterocyclic bases and sugars, thus forming glycoside bonds. In general terms, nucleoside analogs can be prepared by base interchange (transglycosilation reaction) using two different kind of enzymes: nucleoside phosphorylases (NPs or NP) and N-2'-deoxyribosyl transferases (NDTs or NDT). NPs include: Pyrimidine nucleoside phosphorylases (E.C. 2.4.2.1), Purine nucleoside phosphorylases (E.C. 2.4.2.2), Uridine nucleoside phosphorylases (E.C. 2.4.2.3) and Thymidine nucleoside phosphorylases (E.C. 2.4.2.4), according to their substrate specificity for pentofuranose donors and nucleobase acceptors. However, cytosine and its nucleosides (cytidine, 2'-deoxycytidine) or modified cytosinic analogues or derivatives and corresponding nucleosides thereof, are not substrates for these NPs enzymes (Mikhailopulo, I. A, et al. 2010. New Trends in Nucleoside Biotechnology, Acta Naturae, 2(5), 36-58).

Contrarily, N-2'-deoxyribosyl transferases (E.C. 2.4.2.6.) specifically catalyze the direct transfer of the deoxyribofuranosyl moiety between a nucleoside and an acceptor base without intermediary formation of 2-deoxyribofuranose phosphate. NDTs substrate specificity involves strict specificity for the 2-deoxyribofuranose moiety, rather than broad tolerance regarding modified purines and good substrate activity of cytosine as an acceptor of the 2-deoxy- and 2,3-dideoxyribofuranose residues (Mikhailopulo, I. A, et al. 2010. New Trends in Nucleoside Biotechnology, Acta Naturae, 2(5), 36-58). Fresco-Taboada et al (New insights on NDTs: a versatile Biocatalyst for one-pot one-step synthesis of nucleoside analogs, Appl. Microbiol. Biotechnol, 2013, 97, 3773-3785) disclose that cytosine is the best acceptor of 2'-deoxyribofuranose moiety in all cases.

Therefore, the choice of NPs or NDTs depends on the structure of the acceptor-base and the donor nucleoside. NPs and NDTs complement each other, allowing the biocatalytic production of natural nucleoside and their modified analogs, most of them useful for the production of many anticancer and antiviral drugs.

However, none of NPs or NDTs allow the production of cytosinic ribonucleosides, because cytosine is not an acceptor base for NPs while, at the same time, ribofuranose nucleoside donors are not substrates for NDTs. Therefore, the production of cytosinic nucleoside analogues remains a challenge, not only due to the above mentioned substrate specificity, but also because certain enzymes, such as cytosine and cytidine deaminases or cytosine and cytidine deacetylases that are usually present in the biocatalyst preparation, degrade the substrates or the final product, resulting in unproductive methodology for industrial purposes.

Araki et al (EP 1254959, US 7629457) disclosed a method for producing cytosine nucleoside compounds from pentose-1-phosphate and cytosine or a derivative thereof using a nucleoside phosphorylase reactive to cytosine or a bacterium having said enzyme activity. Particularly, the inventors found that a purine nucleoside phosphorylase (PNP) from bacteria belonging to genus *Escherichia,* which itself should catalyze a reaction involving a purine base as substrate instead of a pyridine base, was able to catalyze the production of cytosine nucleoside from cytosine and pentose-1-phosphate. However, the reaction of cytosine or a derivative thereof with pentose-1-phosphate in the presence of the bacterium having the enzyme activity disclosed therein was found to produce almost exclusively the deaminated products of cytosine, or the derivative thereof, thus failing in efficient accumulation of the cytosine nucleoside compound of interest. In order to minimize the production of deaminated cytosine by-products, the referred patents additionally disclosed a method for specifically reducing the deaminase activity that degrade the substrates or the final product, by contacting the enzyme preparation with organic solvents and heating the enzyme preparation at a temperature from 60°C to 90°C for an effective period of time.

Araki et al (US 2005/0074857) disclosed a method for producing a pyrimidine nucleoside compound and a new pyrimidine nucleoside compound using a pyrimidine base derivative using an enzyme known as purine nucleoside phosphorylase (PNP) derived from a microorganism such as *E. coli,* the enzyme also having cytosine nucleoside phosphorylase activity. Authors overcome certain deacetylation deactivation by heating the bacterial cell of the microorganism or the processed enzyme or placing in contact with suitable organic solvents.

The same authors disclosed several methods to produce the above mentioned compounds without applying a complicate deaminase-deactivating treatment, by producing a microorganism lacking both the cytosine deaminase gene and the cytidine deaminase gene (JP2004344029) or by means of a zinc salt (JP2004024086).

Ding Q. 2010. Enzymatic synthesis of nucleosides by mucleoside phosphorylase co-expressed in Escherichia coli. J. Zheijiang Univ-Sci B (Biomed & Biotechnol) 11 (11): 880-888, disclosed synthesis of many types of nucleosides. However, the disclosure failed in producing cytosine or arabinoside nucleosides, therefore away from using NPs for the synthesis of this particular types of nucleosides.

Szeker, K. 2012. Nucleoside phosphorylases from thermophiles, Ph.D. Thesis, Berlin Institute of Biotechnology, Germany, disclose discloses that no phosphorolytic activity of GtPyNP (NP from *Geobacillus thermoglucosidasius),* only poor activity of TtPyNP (MP from *Thermus thermophilus)* were determined for cytidine as substrates (vide supra, p. 84) and cytidine was weakly recognized as substrate by PNPs and ApMTAP (5'-Methylthioadenosine phosphorylase from *Aeropyrum pernix, vide supra,* p. 116).

Thus, the biocatalytic synthesis of cytosinic nucleoside analogues remains a challenge and its application at industrial scale is limited by product degradation due to competitive enzymes that are present in the biocatalyst preparation, as explained above.

Since several non-natural nucleosides acting as antiviral or anticancer agents contain cytosine as nucleobase moiety, it is interesting to explore the development of a novel and effective industrial enzymatic process that may overcome the before mention drawbacks and improve the synthesis of this type of cytosine nucleoside analogues.

Capecitabine, a well known anticancer agent having a cytosine NA chemical structure, it has been produced in the past, according to several processes described in the prior art for the multi-step chemical synthesis of this NA (the originators at F. Hoffmann-La Roche AG, Arasaki et al, EP0602454; Kamiya et al, EP0602478).

The above processes suffer from certain drawbacks such as: the toxic solvents or reagents that are used, the need of a chromatographic purification step, which is unsuitable for large scale production, multi-step complex processes including protection and deprotection stages of the hydroxyl groups on the glycoside subunit, and low to moderate overall yields of capecitabine synthesis. The protection and deprotection sequence adds two extra steps in all these processes, then reducing the overall yield and increasing the time and cost of the process from a commercial production point of view.

In WO 2011/104540 a one step process for the preparation of Capecitabine is disclosed. The process is based on the reaction of 5-fluoro-5'-deoxycytidine with a pentyloxycarbonylation reagent that avoid the need for the conventional protection of the hydroxyl groups of the preformed nucleoside 5-fluoro-5'-deoxycytidine. The reaction takes place in organic solvents, uses chemical catalysts (such as bases or corrosive mineral acids as HCl gas), high temperatures (at solvent reflux, up to 110°C) and long reaction times (usually 9 to 10 hours), rendering Capecitabine in moderate yields (50 to 67%).

The inventors have demonstrated that Capecitabine suffers from thermal instability at high temperatures, and after heating the product at 90°C during 10 hours at neutral pH, up to 40% of the product is lost. Therefore, the process described in WO 2011/104540 suffers from important loses of the final product.

On the contrary, the enzymatic process of the invention runs at milder conditions, using water as solvent and furnishing yields higher than 70%.

Kanan et al (WO 2010/061402) disclose a process using (CALB) Novozyme 435 (a lipase acrylic resin from *Candida antartica*) as catalyst in the presence of organic solvent. Such process requires at least four steps to furnish capecitabine.

Then, according to the above disadvantages associated with the prior art and the importance of capecitabine in the treatment of cancer, there is a great need to develop an improved process for the preparation of this Active Principle Ingredient (API) that does not involve, multiple steps, uses relatively inexpensive reagents and that it would render the nucleoside analogue product in high yield and purity.

The same happens for Cytarabine synthesis. Cytarabine is another well known anticancer agent also sharing a cytosine NA chemical structure, as Capecitabine. There are early patents in the name of Merck (NL6511420 in 1964

or in the name of Salt lake Institute of Biological Studies (1969)), which already described chemical synthesis of said active compound, wherein the same drawbacks mentioned for Capecitabine might be equally mentioned.

Surprisingly, it was found that the drawbacks of previous cited biocatalytic synthesis can be avoided and cytosinic NAs can be obtained with conversion higher than 70% and anomeric purity higher than 95%. That is possible by applying a suitable enzymatic method based on the use of Nucleoside Phosphorylases (NPs), either Purine Nucleoside Phosphorylases (PNPs), or Pyrimidine Nucleoside Phosphorylases (PyNP), or a mixture of Pyrimidine Nucleoside Phosphorylases (PyNP) and Purine Nucleoside Phosphorylases (PNP) enzymes. The referred enzymes, surprisingly, can recognize proper chemically modified cytosines and are able to perform the transglycosilation reaction on donor nucleoside analogs without any evidence of deamination or deacetylation impurities. The inventors have demonstrated that the same biocatalytic reaction but using non modified cytosines as nucleoside acceptor does not render the expected final product. For the purposes of present specification, preferred enzymes to be used in the process of invention are Pyrimidine Nucleoside Phosphorylases. Another preferred embodiment of present invention is the use of a mixture of Pyrimidine Nucleoside Phosphorylases (PyNP) and Purine Nucleoside Phosphorylases (PNP) enzymes, particularly when, as starting compound, a purine nucleoside or derivatives or analogs thereof, are used.

The process of invention applied, as a way of example, to the production of Capecitabine or Cytarabine, as cytosine NAs, has several advantages over the processes used in the prior art for the chemical synthesis of these NAs, and also with regard to the enzymatic synthesis using CALB Novozyme 435, such as:

(i) Reduced number of steps,
(ii) Higher conversions and yields,
(iii) No protection/deprotection strategies for the hydroxyl groups in the sugar are needed,
(iv) Mild reaction conditions: environmentally-friendly technology (water or aqueous medium, neutral pH),
(v) Avoidance of organic solvents in the enzymatic step,
(vi) Extremely good selectivity: stereoselectivity - enantioselectivity, chemo-regioselectivity,
(vii) No side-reactions: impurity profile (reduced by-products content),
(viii) Reduction in overall waste generation,
(ix) Process productivity
(x) Overall lower cost of production

Moreover, there are other additional advantages of the biocatalytic process of invention over the chemical process used in the prior art. Particularly relevant is the absence in the process of the invention of the organic solvents used in the chemical process of Arasaki *et al.* and Kamiya *et al.,* as pyridine, dichloromethane (DCM), acetonitrile (ACN), methanol, tetrahydrofuran (THF), or in the enzymatic process of Kannan *et al* (pyridine, dichloromethane (DCM), etc).

Particularly relevant it is also the absence in the process of the invention of metal catalyst such as stannic chloride, toxic reagents such as sylilating agents, etc. All those process organic solvents and reagents must be removed or destroyed prior to any waste discharge to the environment. A supplementary inconvenient of processes described in the prior art with regard to the procedure of invention is its complexity, as far as processes described in the prior art comprise multi-step procedures including protection and de-protection steps, against the simpler enzymatic process of invention.

## Description of the invention

The process of invention as described herein, using PyNP or PyNP/PNP enzymes, is outlined as follows:

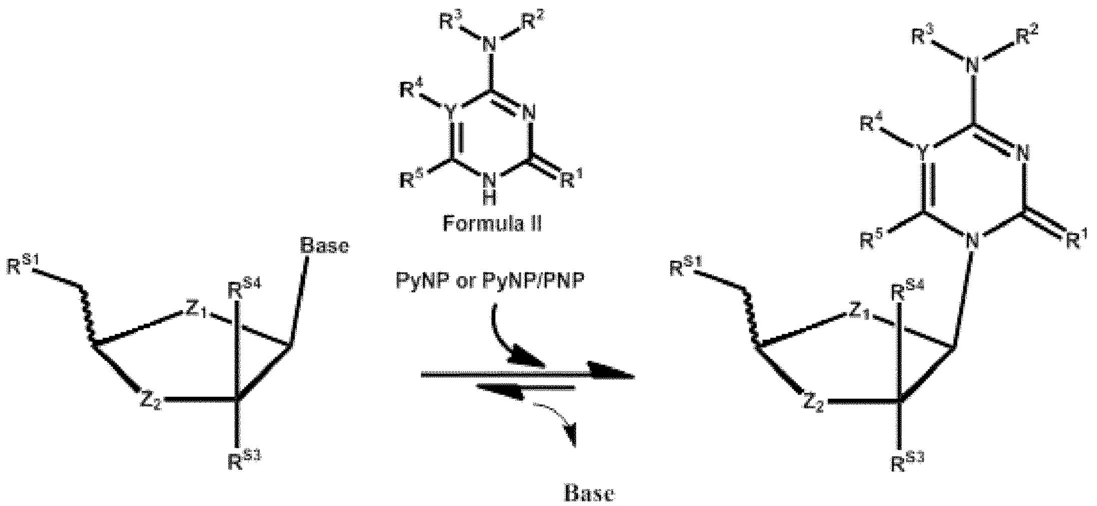


Formula III
(Starting material)

Formula I
(Final product)

Scheme 1. Enzymatic synthesis of cytosinic nucleoside analogs

Applicants have surprisingly found that suitable *N*4-modified cytosine or *N*4-modified cytosine derivatives allow the preparation/production of cytosinic nucleoside analogues at high conversions (more than 70%), completely avoiding side reactions, such as the deamination or deacetylation that are reported in the prior art cited previously, by using nucleoside phosphorylases (either native, recombinant or mutant proteins from bacteria or *archaea*), and particularly using pyrimidine nucleoside phosphorylases (either native, recombinant or mutant proteins from bacteria or *archaea*).

No evidences have been found in the prior art pointing at the fact that a chemical modification/substitution/protection at this position or in any other position in cytosine chemical backbone, would have been able to modify the substrate specificity for nucleoside phosphorylases. For the purposes of present specification the term *N*4-modified cytosine/cytosine derivatives, are synonyms, respectively, of either *N*4-substituted cytosine/cytosine derivatives or *N*4-protected cytosine/cytosine derivatives.

For the purposes of present patent specification the term Cytosine or cytosinic derivatives, nucleosides, intermediates, bases or nucleobases, they all should be understood as chemical compounds derived from cytosine backbone. Particularly as the cytosine or cytosinic derivatives for the purposes of present invention are represented by formula II:

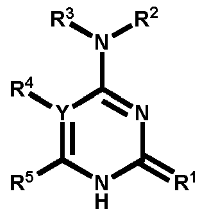

**Formula II**

The suitable chemical modifications that are the object of the present invention provide a more convenient, efficient and easier process for nucleoside analogues synthesis that fully avoids the instability problem related to side reactions. Particularly preferred *N*4-modifications include the modification of the amino group in the form of carbamate (-NHCOO-), more particularly the carbamate being linked to an alkyl chain of 1 to 40 carbon atoms, an alkenyl chain of 1 to 40 carbon atoms or an alkynyl chain of 1 to 40 carbon atoms (the chains being in each case lineal, branched, or substituted by any other functional group), an aryl or and heterocycle. *N*4-modifications of the amino group in the form of amide or related acyl derivatives (-NHCO-) allow the transglycosilation reaction as well, specially when the amido group is being linked to an alkyl chain of 4 to 40 carbon atoms, an alkenyl chain of 1 to 40 carbon atoms or an alkynyl chain of 1 to 40 carbon atoms (the chains being in each case lineal, branched, or substituted by any other functional group), an aryl or and heterocycle. Surprisingly, longer alkyl chains are preferred over short alkyl chains (from 1 o 3 carbon atoms), since according to the experiments carried out, deacetylation side reactions are also observed in short alkyl chains, differing from what US 2005/0074857 teaches away. According to present invention, acyl groups having

an alkyl group of 1 carbon atom do not prevent deacetylation.

More precisely chemical modifications are introduced, according to present description, into cytosine backbone at *N*$^4$ (Nitrogen atom at position 4 in the cytosine heterocycle). More particularly those chemical modifications operated in cytosine skeleton are the ones represented by $R_2$ and/or $R_3$ moieties in formula II:

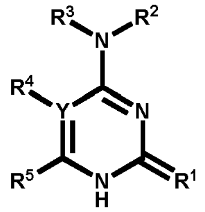

**Formula II**

wherein

$R^1$ being O, $CH_2$, S, NH;

$R^2$ being hydrogen, an optionally substituted $C_{4-40}$ alkyl chain, an optionally substituted alkenyl chain, an optionally substituted alkynyl chain, an optionally substituted aryl linked to N by an optionally substituted alkyl, alkenyl or alkynyl chain, an optionally substituted heterocycle linked to N by an optionally substituted alkyl, alkenyl or alkynyl chain, $COR^6$, $CONR^6R^7$, $CO_2R^6$, $C(S)OR^7$, CN, $SR^6$, $SO_2R^6$, $SO_2R^6$ $R^7$, CN, P(O)aryl, P(O)heterocycle, P(S)aryl, P(S)heterocycle, $P(O)O_2R^8$;

$R^3$ being hydrogen, an optionally substituted $C_{4-40}$ alkyl chain, an optionally substituted alkenyl chain, an optionally substituted alkynyl chain, an optionally substituted aryl linked to N by an optionally substituted alkyl, alkenyl or alkynyl chain, an optionally substituted heterocycle linked to N by an optionally substituted alkyl, alkenyl or alkynyl chain, $COR^6$, $CONR^6R^7$, $CO_2R^6$, $C(S)OR^7$, CN, $SR^6$, $SO_2R^6$; $SO_2R^6$ $R^7$, CN, P(O)aryl, P(O)heterocycle, P(S)aryl, P(S)heterocycle, $P(O)O_2R^8$; being $R^3$ and $R^2$ independent one from each other; and providing that at least one $R_2$ or $R_3$ is different from hydrogen.

$R^4$ being hydrogen, OH, $NH_2$, SH, halogen (preferably F or I); optionally substituted alkyl chain; optionally substituted alkenyl chain; optionally substituted alkynyl chain, trihaloalkyl, $OR^6$, $NR^6R^7$, CN, $COR^6$, $CONR^6R^7$, $CO_2R^6$, $C(S)OR^6$, $OCONR^6R^7$, $OCO_2R^6$, $OC(S)OR^6$, $NHCONR^6R^7$, $NHCO_2R^6$, $NHC(S)OR^6$, $SO_2NR^6R^7$, an optionally substituted aryl linked to Y by an optionally substituted alkyl, alkenyl or alkynyl chain, an optionally substituted heterocycle linked to Y by an optionally substituted alkyl, alkenyl or alkynyl chain, and any optionally substituted heterocycle or optionally substituted aryl of, independently, $R^1$, $R^2$, $R^3$, $R^4$ or $R^5$, selected from:

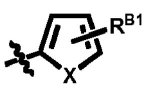 , 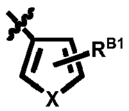 , 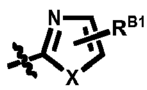 , 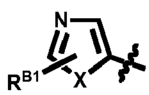 ,

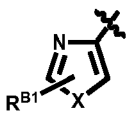 , 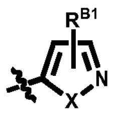 , 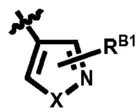 , 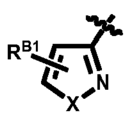 ,

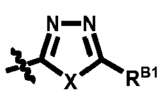 , 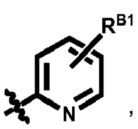 , 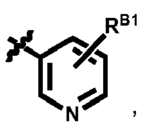 , 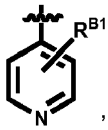 ,

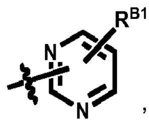, 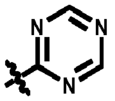, 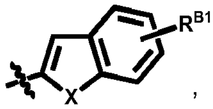, 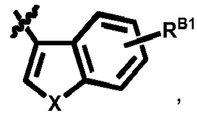,

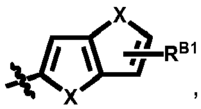, 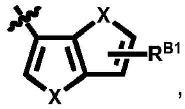, 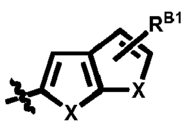, 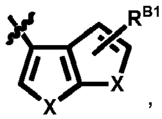,

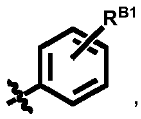,

providing Y is a carbon or sulphur atom and, alternatively, $R^4$ being absent, providing Y is a nitrogen atom;

wherein

X being O, S, N-$R^{B2}$, Se; $R^{B1}$ being H, OH, $NH_2$, straight or branched $C_{1-10}$ alkyl, F, Cl, Br, I, X-$R^{B2}$, -C≡C-$R^{B2}$, $CO_2R^{B2}$; $R^{B2}$ being H, OH, $NH_2$, straight or branched $C_{1-5}$ alkyl, phenyl;

$R^5$ being hydrogen, OH, $NH_2$, SH, halogen (preferably F or I), an optionally substituted alkyl chain, an optionally substituted alkenyl chain, an optionally substituted alkynyl chain, trihaloalkyl, $OR^6$, $NR^6R^7$, CN, $COR^6$, $CONR^6R^7$, $CO_2R^6$, $C(S)OR^6$, $OCONR^6R^7$, $OCO_2R^6$, $OC(S)OR^6$, $NHCONR^6R^7$, $NHCO_2R^6$, $NHC(S)OR^6$, $SO_2NR^6R^7$; $CH_2$-heterocyclic ring, CN;

and any optionally substituted heterocycle or optionally substituted aryl of, independently, $R^2$, $R^3$, $R^4$ or $R^5$, selected from:

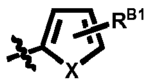, 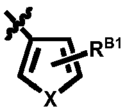, 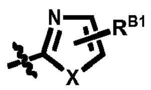, 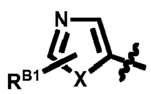,

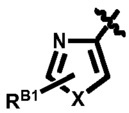, 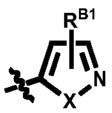, 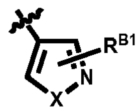, 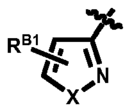,

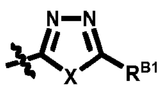, 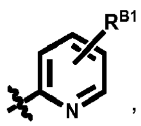, 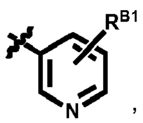, 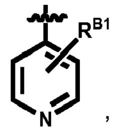,

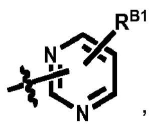, 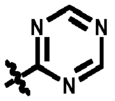, 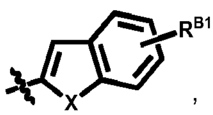, 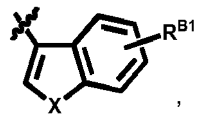,

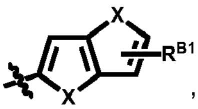, 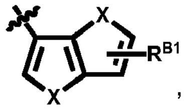, 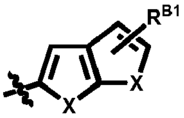, 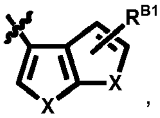,

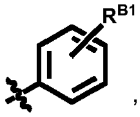,

wherein

X being O, S, N-$R^{B2}$, Se; $R^{B1}$ being H, OH, $NH_2$, SH, straight or branched $C_{1-10}$ alkyl, F, Cl, Br, I, X-$R^{B2}$, -C≡C-$R^{B2}$, $CO_2R^{B2}$; $R^{B2}$ being H, OH, $NH_2$, straight or branched $C_{1-5}$ alkyl, phenyl;

$R^6$ and $R^7$ are independently of each other hydrogen, optionally substituted alkyl chain, optionally substituted alkenyl chain, optionally substituted alkynyl chain, heterocyclic or optionally substituted aryl;

$R^8$ being hydrogen, an optionally substituted alkyl chain, an optionally substituted alkenyl chain, an optionally substituted alkynyl chain, an optionally substituted aryl an optionally substituted heterocycle;

Y being C, N, S;

According to the comparative examples carried out by the applicants and shown in present description, cytosine derivatives that do not contain suitable modifications at *N*4 do not undergo the transglycosilation reaction using nucleoside phosphorylases (native, recombinant or mutant enzymes), particularly using pyrimidine nucleoside phosphorylases, and therefore, do not render the desired nucleoside final product. Moreover, non-protected cytosines (either bases or the corresponding nucleosides) experienced side reactions (such as deamination or deacetylation), that some authors try to deactivate by conventional methods such as heating or using organic solvents (EP1254959 and US2005/0074857), adding zinc salts (JP2004024086) or by using more sophisticated methods, such as using a microorganism simultaneously lacking both the cytosine deaminase gene and the cytidine deaminase gene (JP2004344029). All the above mentioned technical solutions disclosed in the prior art, lowered drastically the overall yield of the corresponding synthesis process. Present invention provides a synthesis method of nucleoside analogues to completely and definitively resolve the above referred technical problems concerning side-reactions that diminished the overall yield of the synthesis process described herein.

The functional group used for modifying/substituting/protecting *N*4 position in the final product obtained, may be optionally removed (deprotection of final products), according to the chemical structure of, specifically, each final product to be produced. However, in certain cases, such as for Capecitabine molecule, there is no need to deprotect the modified/substituted/protected cytosinic *N*4 group, since the modification remains attached to the amino group in the final product (or API). For Capecitabine itself, the enzymatic method of invention shortens, to a large extend, the time needed when the chemical conventional production processes are used.

Hence, the invention provides improved alternative synthesis methods of nucleoside analogues, useful as anticancer and/or antiviral products, by shortening conventional multi-step synthesis, increasing overall yield, reducing side reactions and by-product content and, therefore, improving product purity and quality.

For the purposes of present description, the following terms are further defined as follows.

The term "nucleoside" refers to all compounds in which a heterocyclic base is covalently coupled to a sugar, and especially preferred coupling of the nucleoside to the sugar includes a C1'-(glycosidic) bond of a carbon atom in a sugar to a carbon-or heteroatom (typically nitrogen) in the heterocyclic base. Therefore, in the present context the term "nucleoside" means the glycoside of a heterocyclic base. Similarly, the term "nucleotide" refers to a nucleoside wherein a phosphate group is coupled to the sugar.

The term "nucleoside" may be used broadly as to include non-naturally occurring nucleosides, naturally occurring nucleosides as well as other nucleoside analogues. Illustrative examples of nucleosides are ribonucleosides comprising a ribose moiety as well as deoxyribonucleosides comprising a deoxyribose moiety. With respect to the bases of such nucleosides, it should be understood that this may be any of the naturally occurring bases, e.g. adenine, guanine, cytosine, thymine, and uracil, as well as any modified variants thereof or any possible unnatural bases.

The term "nucleoside analogue", "nucleoside analog", "NA" or "NAs" as used herein refers to all nucleosides in which the sugar is, preferably, not ribofuranose or arabinofuranose and/or in which the heterocyclic base is not a

naturally occurring base (e.g., A, G, C, T, I, etc.).

As further used herein, the term "sugar" refers to all carbohydrates and derivatives thereof, wherein particularly contemplated derivatives include deletion, substitution or addition or a chemical group or atom in the sugar. For example, especially contemplated deletions include 2'-deoxy, 3'-deoxy, 5'-deoxy and/or 2',3'-dideoxy-sugars. Especially contemplated substitutions include replacement of the ring-oxygen with sulphur or methylene, or replacement of a hydroxyl group with a halogen, azido, amino-, cyano, sulfhydryl-, or methyl group, and especially contemplated additions include methylene phosphonate groups. Further contemplated sugars also include sugar analogues (i.e., not naturally occurring sugars), and particularly carbocyclic ring systems. The term "carbocyclic ring system" as used herein refers to any molecule in which a plurality or carbon atoms form a ring, and in especially contemplated carbocyclic ring systems the ring is formed from 3, 4, 5, or 6 carbon atoms.

The term "chemo-enzymatic synthesis" refers to a method of synthesis of chemical compounds through a combination of chemical and biocatalytic steps. For the particular purposes of the present invention, the order of the reaction stages, in one of the preferred embodiments of the invention must be: 1) chemical modification of the cytosinic base; 2) biocatalytic transglycosilation, using a NP enzyme; 3) optionally further deprotection of the cytosine-$N^4$ in the final product.

The term "enzymatic synthesis" refers to a method of synthesis of chemical compounds by means of a process which only comprises biocatalytic steps, carried out by the appropriate enzymes (NPs). Accordingly, other preferred embodiment of the synthesis process described herein is a full biocatalytic process which departs from cytosine derivatives, as the ones previously mentioned as represented by general formula II, already prepared or available in the market as cytosine derivatives as such, particularly those showing $R_2$ and/or $R_3$ at $N^4$ position of cytosine heterocyclic ring, according to present invention, and, therefore, omitting step 1 of chemical modification of the aforesaid cytosine backbone.

The terms "heterocyclic ring" or "heterocyclic base" or "base" or "nucleobase" are used interchangeably herein and refer to any compound in which plurality of atoms form a ring via a plurality of covalent bonds, wherein the ring includes at least one atom other than a carbon atom. Particularly contemplated heterocyclic bases include 5- and 6-membered rings containing at least 1 to 4 heteroatoms each independently selected from nitrogen, oxygen and sulphur as the non-carbon atom (e.g., imidazole, pyrrole, triazole, dihydropyrimidine). Further contemplated heterocycles may be fused (i.e., covalently bound) to another ring or heterocycle, and are thus termed "fused heterocycle" or "fused heterocyclic base" as used herein. Especially contemplated fused heterocycles include a 5-membered ring fused to a 6-membered ring (e.g., purine, pyrrolo[2,3-d]pyrimidine), and a 6-membered ring fused to another 6-membered or higher ring (e.g., pyrido[4,5-d]pyrimidine, benzodiazepine). Examples of these and further preferred heterocyclic bases are given below. Still further contemplated heterocyclic bases may be aromatic, or may include one or more double or triple bonds. Moreover, contemplated heterocyclic bases and fused heterocycles may further be substituted in one or more positions. And any one of the rings being optionally substituted with one, two or three substituents each independently selected from the group consisting of halogen, hydroxy, nitro, cyano, carboxyl, $C_{1-6}$alkyl, $C_{1-6}$alkoxy, $C_{1-6}$alkoxy$C_{1-6}$alkyl, $C_{1-6}$alkylcarbonyl, amino, mono- or di$C_{1-6}$alkylamino, azido, mercapto, polyhalo$C_{1-6}$alkyl, polyhalo$C_{1-6}$alkoxy, and $C_{3-7}$cycloalkyl.

The terms "nucleobase" covers naturally occurring nucleobases as well as non-naturally occurring nucleobases. It should be clear to the person skilled in the art that various nucleobases which previously have been considered "non-naturally occurring" have subsequently found in nature. Thus, "nucleobase" includes not only the known purine and pyrimidine heterocycles, but also heterocyclic analogues (such as *N*-substituted heterocycles) and tautomers thereof. Illustrative examples of nucleobases are adenine, guanine, thymine, cytosine, uracil, purine, xanthine, 2-chloroadenine, 2-fluoroadenine, pentyl (5-fluoro-2-oxo-1,2, dihydropyrimidin-4-yl)carbamate, cytosine *N*-alkyl carbamates, cytosine *N*-alkylesters, 5-azacytosine, 5-bromovinyluracil, 5-fluorouracil, 5-trifluromethyluracil, 6-methoxy-9*H*-purin-2-amine and (*R*)-3,6,7,8-tetrahydroimidazo[4,5-*d*][1,3]diazepin-8-ol.

The term "nucleobase" is intended to cover every and all of these examples as well as analogues and tautomers, and regioisomers thereof. In order to differentiate these "nucleobases" from other heterocyclic bases also present in this specification, for the purposes of present specification, the term "nucleobase" mainly refers to cytosinic bases represented by formula II. However, the term "bases", for the purposes also of present specification, mainly relates to the bases present in the nucleosides represented by formula III.

The term "tautomer" or "tautomeric form" refers to structural isomer of different energies which are interconvertible via a low energy barrier. For example, proton tautomers (also known as prototropic tautomers) include interconversion via migration of a proton, such as keto-enol and imine-enamine isomerizations. Valence tautomers include interconversions by reorganization of some of the bonding electrons.

The term "regioisomer" refers to structural isomer, or constitutional isomer in the sense that refers to molecules with the same molecular formula that whose atoms are bonded in different order of connectivity.

The term "conversion" refers to is the percentage of starting material that is transformed into products, either the expected final product, byproducts, or even into products of degradation.

The term "yield" is the number of synthesized molecules of product per number of starting molecules. In a multistep synthesis, the yield can be calculated by multiplication of the yields of all the single steps.
The term "anomeric purity" refers to the amount of a particular anomer of a compound divided by the total amount of all anomers of that compound present in the mixture multiplied by 100%.
The term "cytosine modification/substitution/protection" refers to any nucleoside analogue in which at least one position on the original backbone of cytosine (except nitrogen at position 1) is substituted by a functional group such as those described as radicals $R^1$, $R^2$, $R^3$, $R^4$, $R^5$, X and/or Y, each one of these groups being independent from the others.
The term "cytosine modified/substituted/protected at position $N^4$" refers to bases with cytosine backbone in which at least one proton of the amino group at position 4 is substituted by a functional group, such as those described as radicals $R_2$ and/or $R^3$, each one of these substituents being independent from the others, providing that at least one of the aforesaid substituents is different from hydrogen.
The term "intermediate" or "intermediates" refer to any nucleoside analogue type compounds which may be transformed into an active pharmaceutical ingredient (API) of nucleosidic structure by means of suitable additional chemical reactions. Therefore, intermediates are molecules that may be considered as API precursors. For the purposes of present specification the term "precursor" when applied to the $N^4$- modified cytosine or cytosine derivatives or intermediates, is meant by compounds of formula II wherein moieties bound to $N^4$ are chemical groups other than those represented by $R^2$ or $R^3$.
The term "prodrug" as used throughout this text means the pharmacologically acceptable derivatives such as esters, amides, carbamates and phosphates, such that the resulting *in vivo* biotransformation product of the derivative is the active drug as defined in the compounds of formula (I). The reference by Goodman and Gilman (The Pharmacological Basis of Therapeutics, 8th ed, McGraw-Hill, Int. Ed. 1992, "Biotransformation of Drugs", p 13-15) describing prodrugs generally is hereby incorporated. Prodrugs preferably have excellent aqueous solubility, increased bioavailability and are readily metabolized into the active inhibitors *in vivo.* Prodrugs of a compound of the present invention may be prepared by modifying functional groups present in the compound in such a way that the modifications are cleaved, either by routine manipulation or *in vivo,* to the parent compound.
Preferred prodrugs are pharmaceutically acceptable ester, amide and carbamate that are hydrolysable *in vivo* and are derived from those compounds of formula I having a hydroxy or a amino group. An *in vivo* hydrolysable ester, amide and carbamate is an ester, amide or carbamate group which is hydrolysed in the human or animal body to produce the parent acid or alcohol. Suitable pharmaceutically acceptable esters, amide and carbamates for amino group include $C_{1-6}$alkoxymethyl esters for example methoxymethyl, $C_{1-6}$ alkanoyloxymethyl esters for example pivaloyloxymethyl, phthalidyl esters, $C_{3-8}$ cycloalkoxycarbonyloxy$C_{1-6}$alkyl esters for example l-cyclohexylcarbonyloxyethyl; 1,3-dioxolen-2-onylmethyl esters for example 5-methyl-l,3-dioxolen-2-onylmethyl; and $C_{1-6}$alkoxycarbonyloxyethyl esters for example 1-methoxycarbonyl-oxyethyl which may be formed at any carboxy group in the compounds of this invention.
An *in vivo* hydrolysable ester of a compound of the formula (I) containing a hydroxy group includes inorganic esters such as phosphate esters and $\alpha$-acyloxyalkyl ethers and related compounds which as a result of the *in vivo* hydrolysis of the ester breakdown to give the parent hydroxy group. Examples of $\alpha$-acyloxyalkyl ethers include acetoxymethoxy and 2,2-dimethylpropionyloxy-methoxy. A selection of *in vivo* hydrolysable ester forming groups for hydroxy include alkanoyl, benzoyl, phenylacetyl and substituted benzoyl and phenylacetyl, alkoxycarbonyl (to give alkyl carbonate esters), dialkylcarbamoyl and N-(dialkylaminoethyl)-N-alkylcarbamoyl (to give carbamates), dialkylaminoacetyl and carboxyacetyl. Examples of substituents on benzoyl include morpholino and piperazino linked from a ring nitrogen atom via a methylene group to the 3- or 4-position of the benzoyl ring.
For therapeutic use, salts of the compounds of formula (I) are those wherein the counter-ion is pharmaceutically acceptable. However, salts of acids and bases which are non-pharmaceutically acceptable may also find use, for example, in the preparation or purification of a pharmaceutically acceptable compound. All salts, whether pharmaceutically acceptable or not are included within the scope of the present invention.
The pharmaceutically acceptable acid and base addition salts as mentioned above are meant to comprise the therapeutically active non-toxic acid and base addition salt forms which the compounds of formula (I) are able to form. The pharmaceutically acceptable acid addition salts can conveniently be obtained by treating the base form with such appropriate acid. Appropriate acids comprise, for example, inorganic acids such as hydrohalic acids, e.g. hydrochloric or hydrobromic acid, sulfuric, nitric, phosphoric and the like acids; or organic acids such as, for example, acetic, propanoic, hydroxyacetic, lactic, pyruvic, oxalic (i.e. ethanedioic), malonic, succinic (i.e. butanedioic acid), maleic, fumaric, malic (i.e. hydroxybutanedioic acid), tartaric, citric, methanesulfonic, ethanesulfonic, benzenesulfonic, p-toluenesulfonic, cyclamic, salicylic, p-aminosalicylic, pamoic and the like acids.
Conversely said salt forms can be converted by treatment with an appropriate base into the free base form.
The compounds of formula (I) containing an acidic proton may also be converted into their non-toxic metal or amine addition salt forms by treatment with appropriate organic and inorganic bases. Appropriate base salt forms comprise, for example, the ammonium salts, the alkali and earth alkaline metal salts, e.g. the lithium, sodium, potassium, magnesium, calcium salts and the like, salts with organic bases, e.g. the benzathine, N-methyl-D-glucamine, hydrabamine

salts, and salts with amino acids such as, for example, arginine, lysine and the like.

The term addition salt as used hereinabove also comprises the solvates which the compounds of formula (I) as well as the salts thereof, are able to form. Such solvates are for example hydrates, alcoholates and the like.

The term "quaternary amine" as used hereinbefore defines the quaternary ammonium salts which the compounds of formula (I) are able to form by reaction between a basic nitrogen of a compound of formula (I) and an appropriate quaternizing agent, such as, for example, an optionally substituted alkylhalide, arylhalide or arylalkylhalide, e.g. methyliodide or benzyliodide. Other reactants with good leaving groups may also be used, such as alkyl trifluoromethanesulfonates, alkyl methanesulfonates, and alkyl p-toluenesulfonates. A quaternary amine has a positively charged nitrogen. Pharmaceutically acceptable counterions include chloro, bromo, iodo, trifluoroacetate and acetate. The counterion of choice can be introduced using ion exchange resins.

The N-oxide forms of the present compounds are meant to comprise the compounds of formula (I) wherein one or several nitrogen atoms are oxidized to the so-called N-oxide.

It will be appreciated that the compounds of formula (I) may have metal binding, chelating, complex forming properties and therefore may exist as metal complexes or metal chelates. Such metalated derivatives of the compounds of formula (I) are intended to be included within the scope of the present invention.

Some of the compounds of formula (I) may also exist in their tautomeric form. Such forms although not explicitly indicated in the above formula are intended to be included within the scope of the present invention.

The term "alkyl" as used herein it does refer to any linear, branched, or cyclic hydrocarbon in which all carbon-carbon bonds are single bonds.

The term "alkenyl" and "unsubstituted alkenyl" are used interchangeably herein and refer to any linear, branched, or cyclic alkyl with at least one carbon-carbon double bond.

Furthermore, the term "alkynyl" as used herein it does refer to any linear, branched, or cyclic alkyl or alkenyl with at least one carbon-carbon triple bond.

The term "aryl" as used herein it does refer to any aromatic cyclic alkenyl or alkynyl, being as a group or part of a group is phenyl or naphthalenyl, each optionally substituted with one, two or three substituents selected from halo, hydroxy, nitro, cyano, carboxyl, $C_{1-6}$alkyl, $C_{1-6}$alkoxy, $C_{1-6}$alkoxy$C_{1-6}$alkyl, $C_{1-6}$alkylcarbonyl, amino, mono- or di$C_{1-6}$alkylamino, azido, mercapto, polyhalo$C_{1-6}$alkyl, and poiyhaio$C_{1-6}$alkoxy. The term "alkaryl" is employed where an aryl is covalently bound to an alkyl, alkenyl, or alkynyl.

The term "substituted" as used herein refers to a replacement of an atom or chemical group (e.g., H, $NH_2$, or OH) with a functional group, and particularly contemplated functional groups include nucleophilic groups (e.g., $-NH_2$, -OH, -SH, -NC, etc.), electrophilic groups (e.g., C(O)OR, C(X)OH, etc.), polar groups (e.g., -OH), non-polar groups (e.g., aryl, alkyl, alkenyl, alkynyl, etc.), ionic groups (e.g., $-NH_3^+$), and halogens (e.g., -F, -Cl), and all chemically reasonable combinations thereof. Thus, the term "functional group" and the term "substituent" are used interchangeably herein and refer to nucleophilic groups (e.g., $-NH_2$, -OH, -SH, -NC, -CN, etc.), electrophilic groups (e.g., C(O)OR, C(X)OH, C(Halogen)OR, etc.), polar groups (e.g., -OH), non-polar groups (e.g., aryl, alkyl, alkenyl, alkynyl, etc.), ionic groups (e.g., $-NH_3^+$), and halogens.

For the purposes of present description, the term "Nucleoside Phosphorylase " or "NP" or "NPs" comprises Pyrimidine Nucleoside Phosphorylase enzymes or PyNP (PyNPs) enzymes or mixtures of PyNPs and PNPs (Purine Nucleoside Phosphorylase enzymes). Those enzymes may be obtained from naturally occurring microorganisms, either mesophiles, thermophiles, hyperthermophiles or extremophiles. For the purpose of the present description organisms or NPs enzymes, mesophiles or mesophilic are those able to work or to carry out a NP activity, at temperatures ranging from 18 to 60°C, with an optimal temperature range of 40-55°C. Organisms or NPs enzymes, thermophiles or thermophilic are those able to work or to carry out a NP activity, at temperatures over 60°C and up to 80°C. Organisms or NPs enzymes, hyperthermophiles or hyperthermophilic are those able to work or to carry out a NP activity, at temperatures over 80°C an up to 100°C, with an optimal temperature range of 80-95°C. Additionally, the enzymes used in present invention may be cloned enzymes, obtained by genetic recombination techniques, expressed in host cells transformed with the corresponding vectors carrying the respective encoding nucleic acid sequences.

The nucleic acid molecule encoding a NP enzyme according to present invention is preferably selected from: SEQ ID NO. 1, 2, 5, 7 , 9 or 11; or

a) a nucleotide sequence which is the complement of SEQ ID. NO:1, 2, 5, 7 , 9 or 11 ; or

b) a nucleotide sequence which is degenerate with SEQ ID. NO:1, 2, 5, 7 , 9 or 11; or

c) a nucleotide sequence hybridizing under conditions of high stringency to SEQ ID. NO:1, 2, 5, 7 , 9 or 11; to the complement of SEQ ID. NO:1, 2, 5, 7 , 9 or 11; or to a hybridization probe derived from SEQ ID. NO:1, 2, 5, 7 , 9 or 11; or their complement thereof ; or

d) a nucleotide sequence having at least 80% sequence identity with SEQ ID. NO:1, 2, 5, 7 , 9 or 11; or

e) a nucleotide sequence having at least 59% sequence identity with SEQ ID. NO:1, 2, 5, 7 , 9 ,or 11,; or

f) a nucleotide sequence encoding for an amino acid sequence selected from: SEQ ID. NO: 3, 4, 6, 8, 10 or 12,

Conditions of stringency hybridization in the sense of the present invention are defined as those described by Sambrook et al., Molecular Cloning, A Laboratory Manual, Cold Spring Harbor Laboratory Press (1989), 1.1011.104. According to this, hybridization under stringent conditions means that a positive hybridization signal is still observed after washing for one hour with 1 x SSC buffer and 0.1 % SDS at 55°C, preferably at 62°C and most preferred at 68°C, in particular, for one hour in 0.2 x SSC buffer and 0.1 % SDS at 55°C, preferably at 62°C and most preferred at 68°C.

Moreover, in the sense of present description, the invention also covers nucleotide or amino acid sequences which, on nucleotide or amino acidic levels, respectively, have an identity of at least 59%, particularly preferred at least 80% and most preferred at least 90% to the nucleotide or amino acid sequence shown in SEQ ID NO. 1 or SEQ ID NO: 2 (nucleotidic) or SEQ ID NO. 3 or SEQ ID NO:4, (amino acidic). Percent identity are determined according to the following equation:

$$I = (n/L) \times 100$$

wherein I are percent identity, L is the length of the basic sequence and n is the number of nucleotide or amino acid difference of a sequence to the basic sequence.

Table-1. Identity of *purine nucleoside phosporylase (deaD) [Sulfolobus solfataricus]* protein sequences with similar PNP proteins in GenBank (http://www.ncbi.nlm.nih.gov/genbank/) on 15.11.2013.

| Accession number | Organism | Identity% |
|---|---|---|
| NP_342951.1 | Sulfolobus solfataricus P2 (SEQ ID NO: 3) | 100% |
| NP_378450.1 | *Sulfolobus tokodaii str. 7* | 83% |
| YP_005648757.1 | *Sulfolobus islandicus LAL 14/1* | 59% |
| WP_016730864.1 | *Sulfolobus islandicus* | 64% |
| WP_016730167.1 | *Sulfolobus islandicus* | 61% |

Table-2. Identity of *uridine phosphorylase [Aeropyrum pernix K1* protein sequences with similar UDP proteins in GenBank (http://www.ncbin.nlm.nih.gov/genbank/), on 15.11.2013.

| Accession number | Organism | Identity% |
|---|---|---|
| NP_148386.2 | Aeropyrum pernix K1 (SEQ ID NO: 4) | 100% |
| YP_008604720.1 | *Aeropyrum camini SY1* | 93% |

Still another subject matter of the present invention is a recombinant vector comprising at least one copy of the nucleic acid molecules as defined above, operatively linked with an expression control sequence. The vector may be any prokaryotic or eukaryotic vector. Examples of prokaryotic vectors are chromosomal vectors such as bacteriophages (e. g. bacteriophage Lambda) and extrachromosomal vectors such as plasmids (see, for example, Sambrook et al., supra, Chapter 1-4). The vector may also be a eukaryotic vector, e. g. a yeast vector or a vector suitable for higher cells, e. g. a plasmid vector, viral vector or plant vector. Suitable eukaryotic vectors are described, for example, by Sambrook *et al., supra,* Chapter 16. The invention moreover relates to a recombinant cell transformed with the nucleic acid or the recombinant vector as described above. The cell may be any cell, e. g. a prokaryotic or eukaryotic cell. Prokaryotic cells, in particular, *E. coli cells,* are especially preferred.

For the purpose of present description, the invention also covers variants, or orthologues of SEQ ID NO: 1 to SEQ ID NO:12.

The term "variant" as used throughout the specification is to be understood to mean a nucleotide sequence of a nucleic acid or amino acid sequence of a protein or polypeptide that is altered by one or more nucleotides or amino

acids, respectively. The variant may have "conservative" changes, wherein a substituted nucleotide or amino acid has similar structural or chemical properties to the replaced nucleotide or amino acid. A variant may also have "non-conservative" changes or a deletion and/or insertion of one or more nucleotides or amino acids. The term also includes within its scope any insertions/deletions of nucleotides or amino acids for a particular nucleic acid or protein or polypeptide. A "functional variant" will be understood to mean a variant that retains the functional capacity of a reference nucleotide sequence or a protein or polypeptide.

The term "complement" or "complementary" as used herein may mean that each strand of 20 double-stranded nucleic acids such as, DNA and RNA, is complementary to the other in that the base pairs between them are non-covalently connected via two or three hydrogen bonds. For DNA, adenine (A) bases complement thymine (T) bases and vice versa; guanine (G) bases complement cytosine (C) bases and vice versa. With RNA, it is the same except that adenine (A) bases complement uracil (U) bases instead of thymine (T) bases. Since there is only one 25 complementary base for each of the bases found in DNA and in RNA, one can reconstruct a complementary strand for any single strand.

The term "orthologue" as used throughout the specification is to be understood as homologous gene or miRNAs sequences found in different species.

A "recombinant DNA molecule" is understood under present specification, as a DNA molecule that has undergone a molecular biological manipulation. The term "recombinant DNA" therefore, it is a form of DNA that does not exist naturally, which is created by combining DNA sequences that would not normally occur together. Once introduced into a host cell, a "recombinant DNA molecule" is replicated by the host cell. By "recombinant protein or enzyme" herein is meant a protein or enzyme produced by a method employing a "recombinant DNA molecule" and hence, also for the purposes of present description, the term recombinant enzyme or recombinant type enzyme should be understood as a protein or enzyme that is derived from recombinant DNA.

Mutant enzyme, for the purposes of present specification is meant by any enzyme which shows at least one mutation, either naturally occurring of induced by human manipulation, including genetic engineering.

On the contrary, native, natural or naturally occurring enzyme, for the purposes of present specification all taken as synonims, is meant by an enzyme which conserves its former amino acid sequence as it is found widespread mostly in nature, without mutations.

By functional part of the enzymes of present invention is meant any sequence fragment of the original enzyme, or of a DNA segement enconding the same, that keeps the ability of the full enzyme sequence to carry out all its biocathalysis properties.

The term cytosinic nucleoside analogues means, for the purposes of present specification, either nucleoside analogues of cytosine or nucleoside analogues of cytosine derivatives. Equally, the term cytosinic base means, as constrained to the present patent specification, the base cytosine or derivatives thereof. Analogously, the term modified cytosinic base, as used in present specification, covers compounds represented by formula II, wherein, the skeleton of cytosine base is substituted in positions 4, 5 or 6.

Present description discloses a enzymatic process for producing cytosinic nucleoside analogues, particularly useful as active pharmaceutical ingredients (APIs), intermediates or prodrugs thereof, being those APIs or their intermediates, nucleoside analogues (NAs) of formula I:

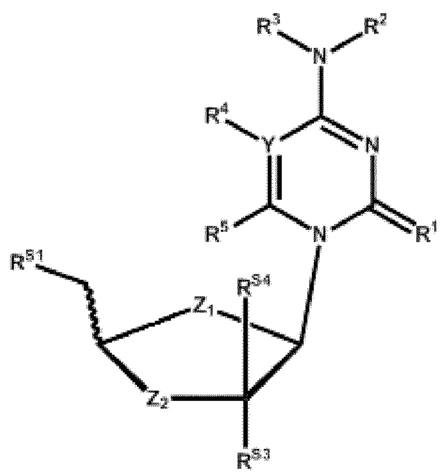

Formula I

wherein,

$Z_1$ being: O, $CH_2$, S, NH;

$Z_2$ being, independently of $Z_1$ O, $C(R^{S2}R^{S5})$, $S(R^{S2}R^{S5})$, $S(R^{S2})$, $S(R^{S5})$, preferably, a group SO or $SO_2$; $N(RS^2R^{S5})$, $N(R^{S2})$, $N(R^{S5})$;

$R^{S1}$ being: hydrogen, OH, ether or ester thereof selected from:

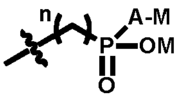 , 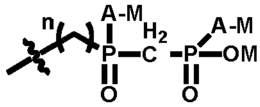 ,

being n is 0 or 1, A is oxygen or nitrogen, and each M is independently hydrogen, an optionally substituted alkyl chain, an optionally substituted alkenyl chain, an optionally substituted alkynyl chain, an optionally substituted aryl optionally linked to P by an optionally substituted alkyl, alkenyl or alkynyl chain, an optionally substituted heterocycle optionally linked to P by an optionally substituted alkyl, alkenyl or alkynyl chain, or a pharmaceutically acceptable counter-ion such as, but not restricted to, sodium, potassium, ammonium or alkylammonium;

$R^{S2}$ being hydrogen, OH or an ether or ester residue thereof, halogen, preferably F, CN, $NH_2$, SH, C≡CH, $N_3$;

$R^{S3}$ being hydrogen, in case of NA derived from 2'-deoxyribouclеosides or arabinonucleosides or being selected from: OH, $NH_2$, halogen, preferably F, $OCH_3$, when the NA is derived from ribonucleosides;

$R^{S4}$ being hydrogen, OH or an ether or ester residue thereof, $NH_2$, halogen, preferably F, CN;

providing $R^{S1}$ and $R^{S4}$ are different when both were ethers or esters of OH residues; $R^{S5}$ being hydrogen, OH or an ether or ester residue thereof, $NH_2$ or halogen, preferably F, when $Z_2$ is different from oxygen;

$R^1$ being O, $CH_2$, S, NH;

$R^2$ being hydrogen, an optionally substituted alkyl chain, preferably $C_{4-40}$alkyl chain, an optionally substituted alkenyl chain, an optionally substituted alkynyl chain, an optionally substituted aryl linked to N by an optionally substituted alkyl, alkenyl or alkynyl chain, an optionally substituted heterocycle linked to N by an optionally substituted alkyl, alkenyl or alkynyl chain, $COR^6$, $CONR^6R^7$, $CO_2R^6$, $C(S)OR^7$, CN, $SR^6$, $SO_2R^6$, $SO_2R^6$ $R^7$, CN, P(O)aryl, P(O)heterocycle, P(S)aryl, P(S)heterocycle, $P(O)O_2R^8$;

$R^3$ being hydrogen, an optionally substituted alkyl chain, preferably $C_{4-40}$ alkyl chain, an optionally substituted alkenyl chain, an optionally substituted alkynyl chain, , an optionally substituted aryl linked to N by an optionally substituted alkyl, alkenyl or alkynyl chain, an optionally substituted heterocycle linked to N by an optionally substituted alkyl, alkenyl or alkynyl chain, $COR^6$, $CONR^6R^7$, $CO_2R^6$, $C(S)OR^7$, CN, $SR^6$, $SO_2R^6$,; $SO_2R^6$ $R^7$, CN, P(O)aryl, P(O)heterocycle, P(S)aryl, P(S)heterocycle, $P(O)O_2R^8$; being $R^3$ and $R^2$ independent one from each other; and providing that at least one $R_2$ or $R_3$ is different from hydrogen.

$R^4$ being hydrogen, OH, $NH_2$, SH, halogen (preferably F or I); optionally substituted alkyl chain; optionally substituted alkenyl chain; optionally substituted alkynyl chain, trihaloalkyl, $OR^6$, $NR^6R^7$, CN, $COR^6$, $CONR^6R^7$, $CO_2R^6$, $C(S)OR^6$, $OCONR^6R^7$, $OCO_2R^6$, $OC(S)OR^6$, $NHCONR^6R^7$, $NHCO_2R^6$, $NHC(S)OR^6$, $SO_2NR^6R^7$, an optionally substituted aryl linked to Y by an optionally substituted alkyl, alkenyl or alkynyl chain, an optionally substituted heterocycle linked to Y by an optionally substituted alkyl, alkenyl or alkynyl chain, and any optionally substituted heterocycle or optionally substituted aryl of, independently, $R^1$, $R^2$, $R^3$, $R^4$ or $R^5$, selected from:

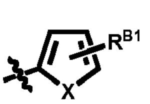 , 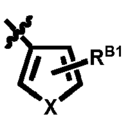 , 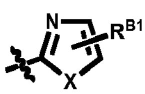 , 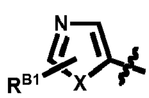 ,

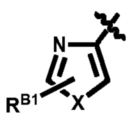 , 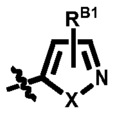 , 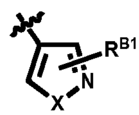 , 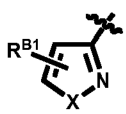 ,

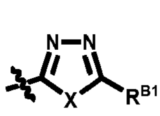 , 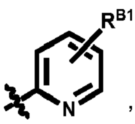 , 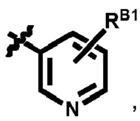 , 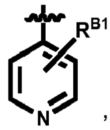 ,

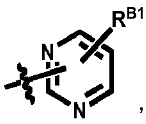, 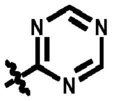, 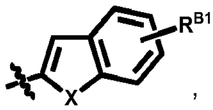, 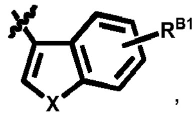,

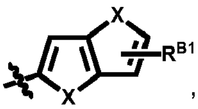, 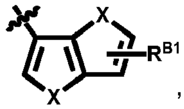, 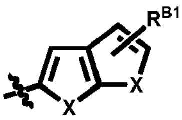, 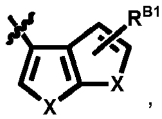,

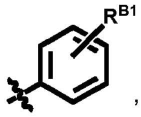,

providing Y is a carbon or sulphur atom and, alternatively, $R^4$ being absent, providing Y is a nitrogen atom; wherein

X being O, S, N-$R^{B2}$, Se; $R^{B1}$ being H, OH, $NH_2$, SH, straight or branched $C_{1-10}$ alkyl, F, Cl, Br, I, X-$R^{B2}$, -C≡C-$R^{B2}$, $CO_2R^{B2}$; $R^{B2}$ being H, OH, $NH_2$, straight or branched $C_{1-5}$ alkyl, phenyl;

$R^5$ being hydrogen, OH, $NH_2$, SH, halogen (preferably F or I), an optionally substituted alkyl chain, an optionally substituted alkenyl chain, an optionally substituted alkynyl chain, trihaloalkyl, $OR^6$, $NR^6R^7$, CN, $COR^6$, $CONR^6R^7$, $CO_2R^6$, $C(S)OR^6$, $OCONR^6R^7$, $OCO_2R^6$, $OC(S)OR^6$, $NHCONR^6R^7$, $NHCO_2R^6$, $NHC(S)OR^6$, $SO_2NR^6R^7$; $CH_2$-heterocyclic ring, CN;

and any optionally substituted heterocycle or optionally substituted aryl of, independently, $R^1$, $R^2$, $R^3$, $R^4$ or $R^5$, selected from:

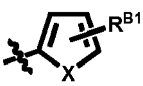, 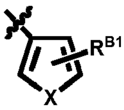, 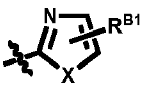, 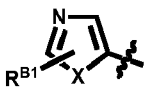,

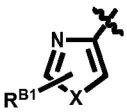, 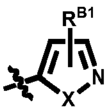, 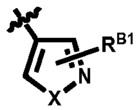, 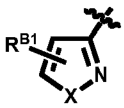,

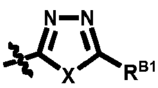, 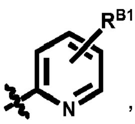, 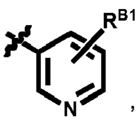, 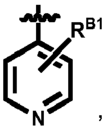,

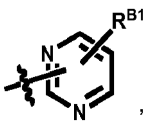, 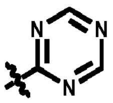, 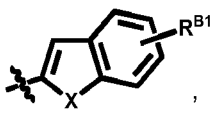, 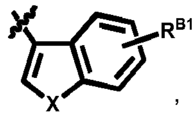,

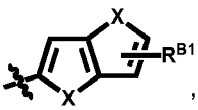, 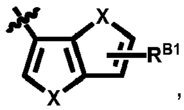, 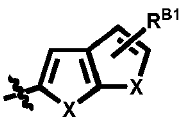, 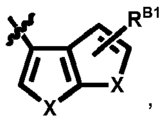,

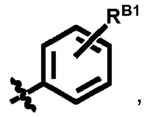
,

wherein
X being O, S, N-$R^{B2}$, Se; $R^{B1}$ being H, OH, $NH_2$, SH, straight or branched $C_{1-10}$ alkyl, F, Cl, Br, I, X-$R^{B2}$, -C≡C-$R^{B2}$, $CO_2R^{B2}$; $R^{B2}$ being H, OH, $NH_2$, straight or branched $C_{1-5}$ alkyl, phenyl;
$R^6$ and $R^7$ are independently of each other hydrogen, optionally substituted alkyl chain, optionally substituted alkenyl chain, optionally substituted alkynyl chain, heterocyclic or optionally substituted aryl;
$R^8$ being hydrogen, an optionally substituted alkyl chain, an optionally substituted alkenyl chain, an optionally substituted alkynyl chain, an optionally substituted aryl an optionally substituted heterocycle;
Y being C, N, S;

wherein the process for producing the cytosinic nucleoside analog comprises the following steps, when the chemo-enzymatic preferred embodiment of the process of the invention was employed:

(i) chemically reacting the cytosinic base with suitable reagents for modifying the amino group at $N^4$ position in cytosine derivative in order to incorporate the proper substitution described as substituent $R^2$ and $R^3$ in intermediate compound of formula II which is optionally purified at the end of step (i) by conventional purification methods; alternatively, the process of invention can depart directly from the starting products represented by formula II (modified cytosinic base), either available in the market or previously synthesized

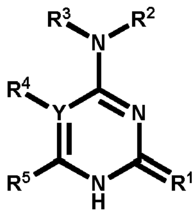
Formula II

(ii) reacting the above mentioned modified cytosinic base with a suitable nucleoside analog starting material, wherein said reaction comprises the addition, in a suitable reaction aqueous medium and under suitable reaction conditions, of a nucleoside phosphorylase enzyme (NPs), preferably a Pyrimidine Nucleoside Phosphorylase enzyme (PyNP), to a mixture of starting materials comprising a cytosine derivative of formula II and a nucleoside analog of formula III

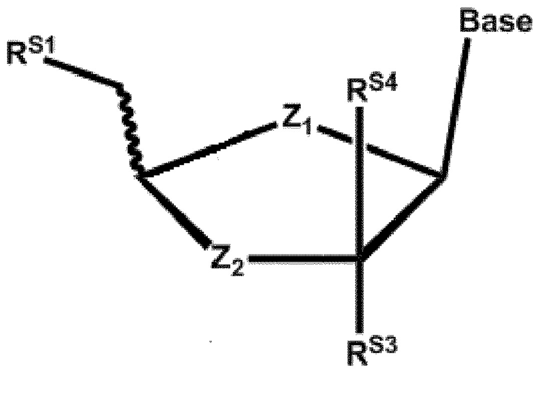
Formula III

wherein,

$Z_1$ being O, $CH_2$, S, NH;
$Z_2$ being, independently of $Z_1$ O, C($R^{S2}R^{S5}$), S($R^{S2}R^{S5}$), S($R^{S2}$), S($R^{S5}$), preferably, a group SO or $SO_2$; N($R^{S2}R^{S5}$), N($R^{S2}$), N($R^{S5}$);
$R^{S1}$ being hydrogen, OH, ether or ester thereof selected from:

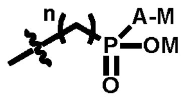 , 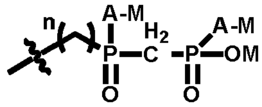 ,

being n is 0 or 1, A is oxygen or nitrogen, and each M is independently hydrogen, an optionally substituted alkyl chain, an optionally substituted alkenyl chain, an optionally substituted alkynyl chain, an optionally substituted aryl optionally linked to P by an optionally substituted alkyl, alkenyl or alkynyl chain, an optionally substituted heterocycle optionally linked to P by an optionally substituted alkyl, alkenyl or alkynyl chain, or a pharmaceutically acceptable counter-ion such as, but not restricted to, sodium, potassium, ammonium or alkylammonium;
$R^{S2}$ being hydrogen, OH or an ether or ester residue thereof, halogen, preferably F, CN, $NH_2$, SH, C≡CH, $N_3$;
$R^{S3}$ being hydrogen, in case of NA derived from 2'-deoxyriboucleosides or arabinonucleosides or being selected from: OH, $NH_2$, halogen (preferably F), $OCH_3$, when the NA is derived from ribonucleosides;
$R^{S4}$ being hydrogen, OH or an ether or ester residue thereof, $NH_2$, halogen, preferably F, CN;
providing $R^{S1}$ and $R^{S4}$ are different when both were ethers or esters of OH residues; $R^{S5}$ being hydrogen, OH or an ether or ester residue thereof, $NH_2$ or halogen, preferably F, in the case that $Z_2$ is different from oxygen;
$R^1$ being O, $CH_2$, S, NH;
$R^2$ being hydrogen, an optionally substituted alkyl chain, preferably $C_{4-40}$alkyl chain, an optionally substituted alkenyl chain, an optionally substituted alkynyl chain, an optionally substituted aryl linked to N by an optionally substituted alkyl, alkenyl or alkynyl chain, an optionally substituted heterocycle linked to N by an optionally substituted alkyl, alkenyl or alkynyl chain, $COR^6$, $CONR^6R^7$, $CO_2R^6$, $C(S)OR^7$, CN, $SR^6$, $SO_2R^6$, $SO_2R^6$ $R^7$, CN, P(O)aryl, P(O)heterocycle, P(S)aryl, P(S)heterocycle, $P(O)O_2R^8$;
$R^3$ being hydrogen, an optionally substituted alkyl chain, preferably $C_{4-40}$alkyl chain, an optionally substituted alkenyl chain, an optionally substituted alkynyl chain, , an optionally substituted aryl linked to N by an optionally substituted alkyl, alkenyl or alkynyl chain, an optionally substituted heterocycle linked to N by an optionally substituted alkyl, alkenyl or alkynyl chain, $COR^6$, $CONR^6R^7$, $CO_2R^6$, $C(S)OR^7$, CN, $SR^6$, $SO_2R^6$,; $SO_2R^6$ $R^7$, CN, P(O)aryl, P(O)heterocycle, P(S)aryl, P(S)heterocycle, $P(O)O_2R^8$; being $R^3$ and $R^2$ independent one from each other; and providing that at least one $R_2$ or $R_3$ is different from hydrogen;
$R^4$ being hydrogen, OH, $NH_2$, SH, halogen (preferably F or I); optionally substituted alkyl chain; optionally substituted alkenyl chain; optionally substituted alkynyl chain, trihaloalkyl, $OR^6$, $NR^6R^7$, CN, $COR^6$, $CONR^6R^7$, $CO_2R^6$, $C(S)OR^6$, $OCONR^6R^7$, $OCO_2R^6$, $OC(S)OR^6$, $NHCONR^6R^7$, $NHCO_2R^6$, $NHC(S)OR^6$, $SO_2NR^6R^7$, an optionally substituted aryl linked to Y by an optionally substituted alkyl, alkenyl or alkynyl chain, an optionally substituted heterocycle linked to Y by an optionally substituted alkyl, alkenyl or alkynyl chain, and any optionally substituted heterocycle or optionally substituted aryl of, independently, $R^2$, $R^3$, $R^4$ or $R^5$, selected from:

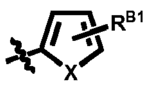 , 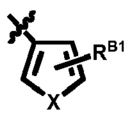 , 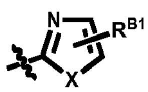 , 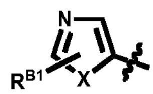 ,

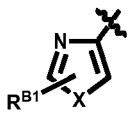 , 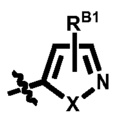 , 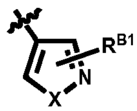 , 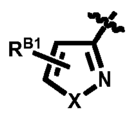 ,

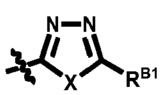 , 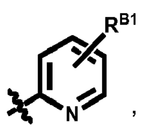 , 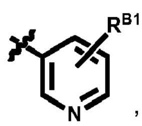 , 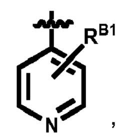 ,

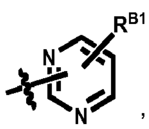 , 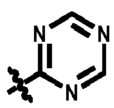 , 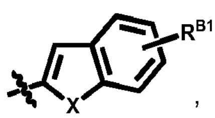 , 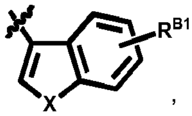 ,

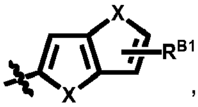 , 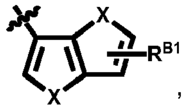 , 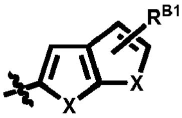 , 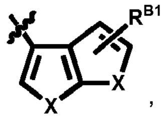 ,

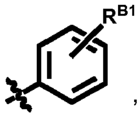 ,

providing Y is a carbon or sulphur atom and, alternatively, $R^4$ being absent, providing Y is a nitrogen atom; wherein

X being O, S, N-$R^{B2}$, Se; $R^{B1}$ being H, OH, $NH_2$, SH, straight or branched $C_{1-10}$ alkyl, F, Cl, Br, I, X-$R^{B2}$, -C≡C-$R^{B2}$, $CO_2R^{B2}$; $R^{B2}$ being H, OH, $NH_2$, straight or branched $C_{1-5}$ alkyl, phenyl;

$R^5$ being hydrogen, OH, $NH_2$, SH, halogen (preferably F or I), an optionally substituted alkyl chain, an optionally substituted alkenyl chain, an optionally substituted alkynyl chain, trihaloalkyl, $OR^6$, $NR^6R^7$, CN, $COR^6$, $CONR^6R^7$, $CO_2R^6$, $C(S)OR^6$, $OCONR^6R^7$, $OCO_2R^6$, $OC(S)OR^6$, $NHCONR^6R^7$, $NHCO_2R^6$, $NHC(S)OR^6$, $SO_2NR^6R^7$; $CH_2$-heterocyclic ring, CN;

and any optionally substituted heterocycle or optionally substituted aryl of, independently, $R^2$, $R^3$, $R^4$ or $R^5$, selected from:

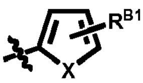 , 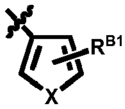 , 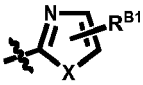 , 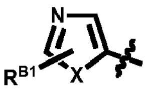 ,

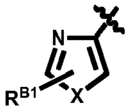 , 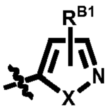 , 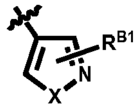 , 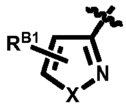 ,

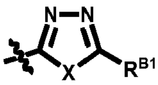 , 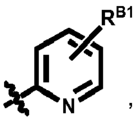 , 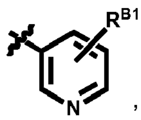 , 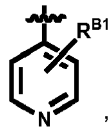 ,

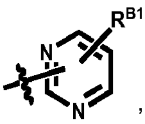 , 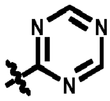 , 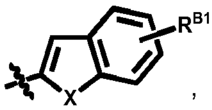 , 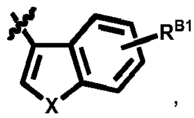 ,

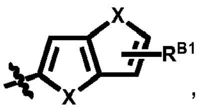 , 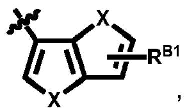 , 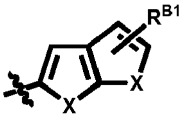 , 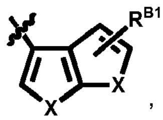 ,

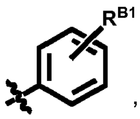 ,

wherein

X being O, S, N-$R^{B2}$, Se; $R^{B1}$ being H, OH, $NH_2$, SH, straight or branched $C_{1-10}$ alkyl, F, Cl, Br, I, X-$R^{B2}$, -C≡C-$R^{B2}$, $CO_2R^{B2}$; $R^{B2}$ being H, OH, $NH_2$, straight or branched $C_{1-5}$ alkyl, phenyl;

$R^6$ and $R^7$ are independently of each other hydrogen, optionally substituted alkyl chain, optionally substituted alkenyl chain, optionally substituted alkynyl chain, heterocyclic or optionally substituted aryl;
$R^8$ being hydrogen, an optionally substituted alkyl chain, an optionally substituted alkenyl chain, an optionally substituted alkynyl chain, an optionally substituted aryl an optionally substituted heterocycle;
Y being C, N, S;

(iii) optionally, deprotecting the amino group at $N^4$ position in cytosinic nucleoside analogue in order to recover the free primary amino $N^4$ at the cytosinic nucleoside analogue of formula I which was further purified by conventional purification methods.

Preferably the heterocyclic ring constituting the base in formula III of the starting materials is selected from: uracil, adenine, cytosine, guanine, thymine, hypoxanthine, xanthine, thiouracil, thioguanine, 9-H-purine-2-amine, 7-methylguanine, 5-fluorouracil, 5-bromouracil, 5-chlorouracil, 5,6-dihydrouracil, 5-methylcytosine and 5-hydroxymethylcytosine and any substituted derivatives thereof.

Moreover, the free cytosine or cytosine derivative to be modified in order to obtain the corresponding nucleobase of formula II is preferably selected from:

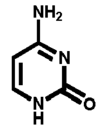

**4-aminopyrimidin-2(1*H*)-one (cytosine)**

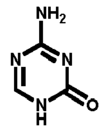

**4-amino-1,3,5-triazin-2(1*H*)-one (5-azacytosine)**

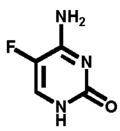

**4-amino-5-fluoropyrimidin-2(1*H*)-one (5-fluorocytosine)**

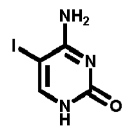

**4-amino-5-iodopyrimidin-2(1*H*)-one (5-iodocytosine)**

Moreover, the cytosinic nucleobase in the nucleoside analog of formula I is preferably selected from:

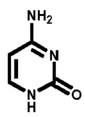

4-aminopyrimidin-2(1*H*)-one (cytosine)

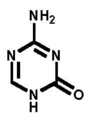

4-amino-1,3,5-triazin-2(1*H*)-one (5-azacytosine)

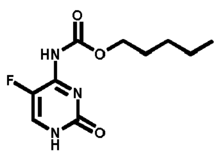

pentyl (5-fluoro-2-oxo-1,2-dihydropyrimidin -4-yl)carbamate

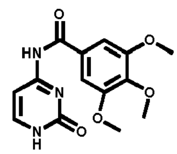

3,4,5-trimethoxy-*N*-(2-oxo-1,2-dihydro-pyrimidin-4-yl)benzamide

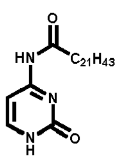

*N*-(2-oxo-1,2-dihydropyrimidin-4-yl) docosanamide

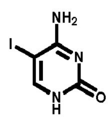

4-amino-5-iodopyrimidin-2(1*H*)-one (5-iodocytosine)

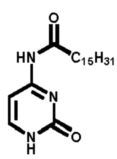

*N*-(2-oxo-1,2-dihydropyrimidin-4-yl) palmitamide

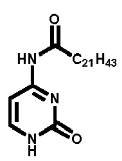

*N*-(2-oxo-1,2-dihydropyrimidin-4-yl) heneicosamide

With the process described herein, the APIs, intermediates or prodrugs thereof produced are selected from: Capecitabine, Decitabine (aza-dCyd or DAC), 5-Azacytidine (aza-Cyd), Cytarabine (ara-C), Enocitabine (BH-AC), Gemcitabine (dFdC), Zalcitabine (ddC), Ibacitabine, Sapacitabine, 2'-C-cyano-2'-deoxy-1-β-D-arabino-pentofuranosylcytosine (CNDAC), Galocitabine, Valopricitabine (NM283), 2'-Deoxy-4'-thiocytidine, Thiarabine (T-araC), 2'-Deoxy-4'-thio-5-azacytidine or Apricitarabine (ATC).

More preferably, the APIs, intermediates or prodrugs thereof produced are selected from: Capecitabine, Decitabine (aza-dCyd or DAC), 5-Azacytidine (aza-Cyd), Cytarabine (ara-C), and still more preferably the described process is particularly intended to the industrial production of Capecitabine or Cytarabine.

Still in one more preferred embodiment of practicing the process describe hereto, the API, intermediates or

prodrugs thereof, produced is Capecitabine (Scheme 2).

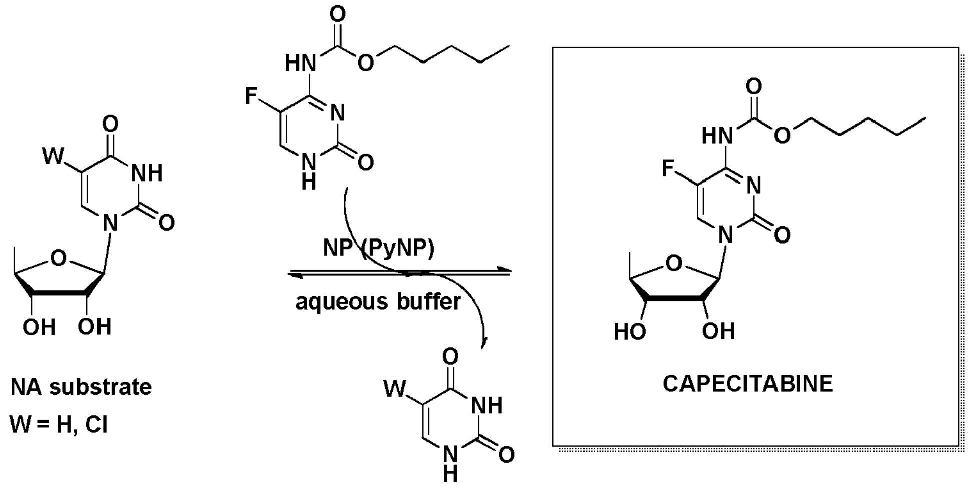


Scheme 2. Synthesis of Capecitabine by NPs or PyNPs

Biocatalytic steps (enzymatic steps) of Capecitabine either enzymatic or chemo-enzymatic synthesis were carried out at temperatures preferably ranging 20-120°C.

Still in one more preferred embodiment of practicing the process describe hereto, the API, intermediates or prodrugs thereof, produced is Cytarabine (Scheme 3).

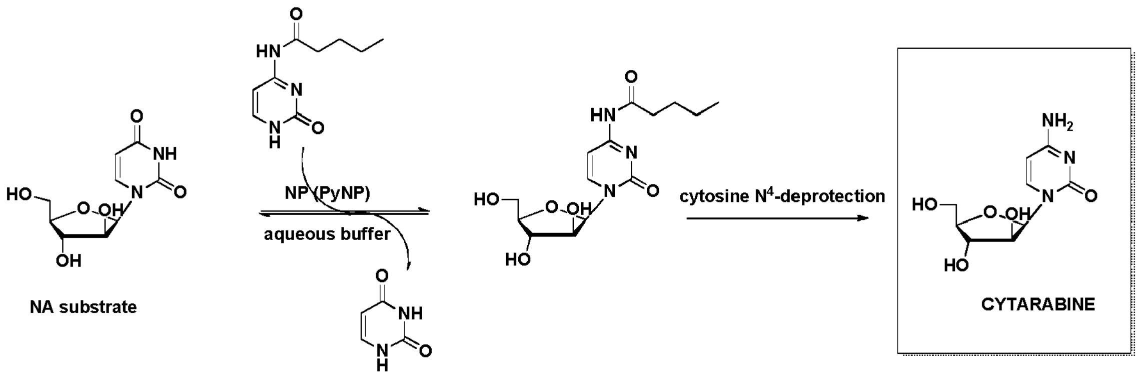


Scheme 3. Synthesis of Cytarabine by NPs or PyNPs

For the purposes of carrying out the biocatalytic process of present description, the applicant has chosen organisms containing NPs enzymes or NPs enzymes isolated as such, preferably, in both cases, PyNP enzymes or PyNP/PNP mixtures of enzymes, wherein either the enzymes or the microorganisms containing them, were mesophiles or mesophilic, in the sense that they were able to work and to carry out a nucleoside transferase activity, at temperatures ranging from 18 to up 60°C, with an optimal temperature range of 40-55°C. Organisms or NPs enzymes, particularly PyNP enzymes, thermophiles or thermophilic are those able to work or to carry out a nucleoside transferase activity, at temperatures ranging over 60°C and up to 80°C. Organisms or NPs enzymes, particularly PyNP enzymes, hyperthermophiles or hyperthermophilic, are those able to work or to carry out a nucleoside transferase activity, at temperatures over 80°C and up to 100°C, with an optimal temperature range of 80-95°C.

The NPs (PyNP) enzyme used in the process of invention can be isolated from a microorganism selected from mesophilic organisms, as a way of example, bacteria, particularly from *E. coli* or from mesophilic, thermophilic or hyperthermophilic *Archaea* particularly from Thermoprotei class and more particularly selected from the genus and species disclosed in WO2011/076894. Even more preferred nucleoside phosphorylases, according to present invention are from

*Sulfolobus solfataricus* and *Aeropyrum pernix.*

Still another embodiment of present invention relates to the use in the process disclosed of a recombinant NP enzyme comprising an amino acid sequence encoded by a nucleic acid sequence, or fragments thereof, selected from SEQ ID NO: 1 or SEQ ID NO: 2. Both DNA sequences are isolated from *Archaea* and, more particularly, from *Archaea* having homologous sequences to nucleoside phosphorylase enzymes.

As described all along present description the preferred embodiment for practicing the process of invention is based in the use as biocatalyst of mesophilic, thermophilic or hyperthermophilic NPs enzymes, either Purine NPs (PNPs), or Pyrimidine NPs (PyNPs), or mistures thereof, for performing the base transfer one step-one pot reaction. Preferably thermophilic or hyperthermophilic NPs enzymes are of recombinant type, comprising gene sequences of fragments thereof, encoding for NP enzymatic activities able to carry out nucleobase transfer one step-one pot reactions, at temperatures ranging over 60°C and up to 100°C and, more preferably, at the suitable reaction conditions described herein.

For the purposes of present description preferred enzymes to be used in the biocatalytic process of invention, methods of cloning the same, vectors carrying the nucleic acid sequences encoding the same and host cells transformed with the aforesaid vectors, all are those disclosed in WO2011/076894, in the name of some of the inventors of present invention and whose patent application whole specification is enclosed herein in full, by reference.

Suitable conditions for carrying out the different embodiments of the process of invention comprise:

a) Temperature ranging 20-120°C
b) Reaction time ranging 1-1000 h
c) Concentration of starting material ranging 1-1000 mM
d) Stoichiometry nucleoside starting material:nucleobase ranges from 1:5 to 5:1.
e) Amount of NP enzyme ranging 0,001-100 mg/ml
f) Nucleobase added to the reaction medium, optionally dissolved in an organic solvent
g) Aqueous reaction medium optionally also containing up to 40% of a suitable organic solvent. Preferably up to 20% and more preferably up to 5%.

Optionally, organic solvents could be added to the reaction medium or be used for dissolving previously the nuclebases. Preferred are polar aprotic solvents, preferably selected from: tetrahydrofuran, acetonitrile, acetone, dimethylformamide (DMF) or dimethylsulfoxide (DMSO).

The process according to present invention also includes an isolation and/or purification steps of the NA produced by standard operation means selected from: precipitation, filtration, concentration or crystallization.

Thermophilic NP enzymes are able to operate at higher temperatures which render the nucleobase transfer reaction more efficient in terms of time and overall yield.

The process of the invention, specifically disclosed as embodiment of the invention, the production of Capecitabine (Scheme 2), wherein the ribonucleoside used as starting material is 5'-deoxyribofuranosyl nucleoside selected from: 5'-deoxyuridine, 5'-deoxy-5-methyluridine or 5'-chloro-5'-deoxyuridine; the nucleobase used as starting material to be transferred by the PyNP enzyme, is pentyl (5-fluoro-2-oxo-1,2-dihydropyrimidin-4-yl)carbamate and the PyNP is a naturally occurring mesophilic, thermophilic or hyperthermophilic enzyme, isolated from bacteria.

One alternative embodiment of the process of invention is the one wherein the API produced is, Capecitabine (Scheme 2), the ribonucleoside used as starting material is 5'-deoxyuridine, 5'-deoxy-5-methyluridine or 5'-chloro-5'-deoxyuridine, the nucleobase also used as starting material to be transferred by the PyNP enzyme, is pentyl (5-fluoro-2-oxo-1,2-dihydropyrimidin-4-yl)carbamate and the PyNP is a recombinant mesophilic, thermophilic or hyperthermophilic enzyme, whose cloned DNA was isolated from bacteria.

A further embodiment of the process of invention is the one wherein the API produced is, Capecitabine (Scheme 2), the ribonucleoside used as starting material is 5'-deoxyuridine, 5'-deoxy-5-methyluridine or 5'-chloro-5'-deoxyuridine, the nucleobase also used as starting material to be transferred by the PyNP enzyme, is pentyl (5-fluoro-2-oxo-1,2-dihydropyrimidin-4-yl)carbamate and the PyNP is a naturally occurring mesophilic, thermophilic or hyperthermophilic enzyme, isolated from *Archaea.*

Still another embodiment of the process of invention is the one wherein the API produced is, Capecitabine (Scheme 2), the ribonucleoside used as starting material is 5'-deoxyuridine, 5'-deoxy-5-methyluridine or 5'-chloro-5'-deoxyuridine, the nucleobase also used as starting material to be transferred by the PyNP enzyme, is pentyl (5-fluoro-2-oxo-1,2-dihydropyrimidin-4-yl)carbamate and the PyNP is a recombinant mesophilic, thermophilic or hyperthermophilic enzyme, isolated from *Archaea.*

The process of the invention, also is represented by the one wherein the API produced is Cytarabine (Scheme 3), the ribonucleoside used as starting material is arabinofuranosyluracil or arabinonucleosides, the nucleobase also used as starting material to be transferred by the PyNP enzyme, is N-(2-oxo-1,2-dihydropyrimidin-4-yl)pentanamide and the PyNP is a naturally occurring mesophilic, thermophilic or hyperthermophilic enzyme, isolated from bacteria.

Another one of the embodiments of the process of invention is that wherein the API produced is Cytarabine

(Scheme 3), the ribonucleoside used as starting material is arabinofuranosyluracil or arabinonucleosides, the nucleobase also used as starting material to be transferred by the PyNP enzyme, is N-(2-oxo-1,2-dihydropyrimidin-4-yl)pentanamide and the PyNP is a recombinant mesophilic, thermophilic or hyperthermophilic enzyme, whose cloned DNA was isolated from bacteria.

An additional embodiments of the process of invention is the one wherein the API produced is, Cytarabine (Scheme 3), the ribonucleoside used as starting material is arabinofuranosyluracil or arabinonucleosides, the nucleobase also used as starting material to be transferred by the PyNP enzyme, N-(2-oxo-1,2-dihydropyrimidin-4-yl)pentanamide and the PyNP is a naturally occurring mesophilic, thermophilic or hyperthermophilic enzyme, whose cloned DNA was isolated from *Archaea.*

Another embodiments of the process of invention is the one wherein the API produced is, Cytarabine (Scheme 3), the ribonucleoside used as starting material is arabinofuranosyluracil or arabinonucleosides, the nucleobase also used as starting material to be transferred by the PyNP enzyme, N-(2-oxo-1,2-dihydropyrimidin-4-yl)pentanamide and the PyNP is a recombinant mesophilic, thermophilic or hyperthermophilic enzyme, whose cloned DNA was isolated from *Archaea.*

Forming part of the same inventive concept, present description also discloses recombinant nucleoside phosphorylase enzymes (either PNPs or PyNPs), for use in any of the processes of the invention as described above. The said native or recombinant NP enzymes can be isolated from mesophilic organisms more preferably, bacteria and *Archaea;* or from thermophilic organisms more preferably, bacteria and *Archaea;* or hyperthermophilic organisms, more preferably bacteria and *Archaea,* and more particularly from *Sulfolobus solfataricus* and *Aeropyrum pernix.*

Also in the same line of integrating a single inventive linked concept, present description also discloses the use of a mesophilic, thermophilic or hyperthermophilic nucleoside phosphorylase (NP o PyNP) in the production of APIs, intermediates or prodrugs thereof, being those APIs, their intermediates or their prodrugs, cytosine nucleoside analogues (NAs) useful as anti-cancer or anti-viral medicaments. More preferably, the previously mentioned use is achieved by the production process and variants thereof, also previously detailed, of NAs as APIs, intermediates or prodrugs thereof. Particularly, related to that previously mentioned use, recombinant thermophilic nucleoside phosphorylase (PNPs, PyNPs or mixtures thereof) are preferred, in the production of APIs, being those APIs or their intermediates, nucleoside analogues (NAs) particularly useful as anti-cancer or anti-viral medicaments.

Among the APIs, intermediates or prodrugs thereof produced by such uses the following may be found:

Capecitabine, Decitabine (aza-dCyd or DAC), 5-Azacytidine (aza-Cyd), Cytarabine (ara-C), Enocitabine (BH-AC), Gemcitabine (dFdC), Zalcitabine (ddC), Ibacitabine, Sapacitabine, 2'-C-cyano-2'-deoxy-1-β-D-arabino-pentofuranosylcytosine (CNDAC), Galocitabine, Valopricitabine (NM283), 2'-Deoxy-4'-thiocytidine, Thiarabine (T-araC), 2'-Deoxy-4'-thio-5-azacytidine or Apricitarabine (ATC).

More preferably, the APIs, intermediates or prodrugs thereof produced by the use of the enzymes disclosed herein, are selected from: Capecitabine, Decitabine (aza-dCyd or DAC), 5-Azacytidine (aza-Cyd), Cytarabine (ara-C), and still more preferably the described process is particularly intended to the industrial production of Capecitabine or Cytarabine and, respectively, intermediates or prodrugs thereof. Also included in present invention are recombinant expression vectors comprising sequences encoding a nucleoside phosphorylase enzymatic activity (NPs) operably linked to one or more control sequences that direct the expression or overexpression of said nucleoside phosphorylases in a suitable host. A preferred recombinant expression vector according to present invention is any carrying and expressing or overexpressing genes encoding NP enzymatic activities existing in thermophilic and hyperthermophilic *Archaea,* preferably *Crenarchaeote* and more preferably from the class *Thermoprotei,* such as: A1 RW90 (A1 RW90THEPD), for the hypothetical protein from Thermofilum pendens (strain Hrk 5); Q97Y30 (Q97Y30SULSO), for the hypothetical protein from Sulfolobus solfataricus; A3DME1 (A3DME1 STAMF), for the hypothetical protein from Staphylothermus marinus (strain ATCC 43588 / DSM 3639 / FI); Q9YA34 (Q9YA34AERPE), for the hypothetical protein from Aeropyrum pernix; A2BJ06 (A2BJ06HYPBU) for the hypothetical protein from Hyperthermus butylicus (strain DSM 5456 / JCM 9403); and D9PZN7 (D9PZN7ACIS3) for the hypothetical protein from Acidilobus saccharovorans (strain DSM 16705 / VKM B-2471 / 345)

Table 3: Encoded NP enzymes

| Acces No. Uniprot Genbank (NCBI) | Entry name | Protein names | Organism | Gene names | SEQ ID NO (DNA/Protein) |
|---|---|---|---|---|---|
| A1 RW90 YP 919473.1. | A1RW90_THEPD | **Purine-nucleoside phosphorylase** | Thermofilum pendens (strain Hrk 5) | Tpen_0060 | 5/6 |
| Q97Y30 NP 342951.1 | Q97Y30_SULSO | **Purine nucleoside phosporylase (DeoD)** | Sulfolobus solfataricus (strain ATCC 35092 / DSM 1617 / JCM 11322 / P2) | deoD SSO 1519 | 1/3 |
| A3DME1 YP 001040709.1. | A3DME1_STAMF | **Purine-nucleoside phosphorylase** | Staphylothermus marinus (strain ATCC 43588 / DSM 3639 / F1) | Smar_0697 | 7/8 |
| Q9YA34 NP 148386.2 | Q9YA34_AERPE | **Uridine phosphorylase** | Aeropyrum pernix (strain ATCC 700893 / DSM 11879 / JCM 9820 / NBRC 100138/K1) | udp APE_2105.1 | 2/4 |
| A2BJ06 YP 001012312.1 | A2BJ06_HYPB U | **Uridine phosphorylase** | Hyperthermus butylicus (strain DSM 5456 / JCM 9403) | Hbut_0092 | 9/10 |
| D9PZN7 YP 003815556.1 | D9PZN7 _ACIS3 | **Uridine phosphorylase** | Acidilobus saccharovorans (strain DSM 16705 / VKM B-2471 / 345-15) | ASAC_0117 | 11/12 |

Preferred recombinant expression vectors according to present invention are carrying and expressing or overexpressing nucleic acid sequence selected from: SEQ ID NO. 1,2,5,7,9 or 11; or

a) a nucleotide sequence which is the complement of SEQ ID. NO:1, 2, 5, 7 , 9 or 11 ; or

b) a nucleotide sequence which is degenerate with SEQ ID. NO:1, 2, 5, 7 , 9 or 11 ; or

c) a nucleotide sequence hybridizing under conditions of high stringency to SEQ ID. NO:1, 2, 5, 7 , 9 or 11; to the complement of SEQ ID. NO:1, 2, 5, 7 , 9 or 11; or to a hybridization probe derived from SEQ ID. NO:1, 2, 5, 7 , 9 or 11; or their complement thereof ; or

d) a nucleotide sequence having at least 80% sequence identity with SEQ ID. NO:1,2,5,7,9 or 11; or

e) a nucleotide sequence having at least 59% sequence identity with SEQ ID. NO:1, 2, 5, 7 , 9 or 11, ; or

f) a nucleotide sequence encoding for an amino acid sequence selected from: SEQ ID. NO: 3, 4, 6, 8, 10 or 12,

The invention also covers the use of the recombinant expression vectors previously mentioned, for the production either of recombinant NPs or for the production of active pharmaceutical ingredients (APIs), intermediates or prodrugs thereof, being those APIs or their intermediates, nucleoside analogues (NAs) particularly useful as anti-cancer or anti-viral medicaments. Particularly APIs, intermediates or prodrugs thereof produced by the aforesaid use are selected from: Capecitabine, Decitabine (aza-dCyd or DAC), 5-Azacytidine (aza-Cyd), Cytarabine (ara-C), Enocitabine (BH-AC), Gemcitabine (dFdC), Zalcitabine (ddC), Ibacitabine, Sapacitabine, 2'-C-cyano-2'-deoxy-1-β-D-arabino-pentofuranosylcytosine (CNDAC), Galocitabine, Valopricitabine (NM283), 2'-Deoxy-4'-thiocytidine, Thiarabine (T-araC), 2'-Deoxy-4'-thio-5-azacytidine and Apricitarabine (ATC).

More preferably, the APIs, intermediates or prodrugs thereof produced are selected from: Capecitabine, Decitabine (aza-dCyd or DAC), 5-Azacytidine (aza-Cyd), Cytarabine (ara-C); and still more preferably the expression vectors described herein are particularly suitable for industrial production of Capecitabine or Cytarabine, intermediates or prodrugs thereof.

More preferably, the previously mentioned use for the production of APIs, intermediates or prodrugs thereof is achieved by the production process and variants thereof, also previously detailed.

The invention also covers host cells comprising the recombinant expression vectors, previously described, particularly when said host cell is *Escherichia coli.* Included as part of the same inventive single linked concept, the use of host cells comprising the recombinant expression vectors as previously described, for the production of recombinant NPs (o PyNP), is also contemplated. Analogously, the use of host cells comprising the recombinant expression vectors as previously described, for the production of active pharmaceutical ingredients (APIs), intermediates or prodrugs thereof, being those APIs or their intermediates, nucleoside analogues (NAs) useful as anti-cancer or anti-viral medicaments, is also part of present invention. Particularly if those host cells comprising the recombinant expression vectors as previously described, are used for producing APIs, intermediates or prodrugs thereof selected from: Capecitabine, Decitabine (aza-dCyd or DAC), 5-Azacytidine (aza-Cyd), Cytarabine (ara-C), Enocitabine (BH-AC), Gemcitabine (dFdC), Zalcitabine (ddC), Ibacitabine, Sapacitabine, 2'-C-cyano-2'-deoxy-1-β-D-arabino-pentofuranosylcytosine (CNDAC), Galocitabine, Valopricitabine (NM283), 2'-Deoxy-4'-thiocytidine, Thiarabine (T-araC), 2'-Deoxy-4'-thio-5-azacytidine and Apricitarabine (ATC).

More preferably, the APIs, intermediates or prodrugs thereof produced using the host cells of present invention, transformed with the recombinant expression vectors, previously described, are selected from: Capecitabine, Decitabine (aza-dCyd or DAC), 5-Azacytidine (aza-Cyd), Cytarabine (ara-C); and still more preferably the host transformed cells described herein are particularly suitable for industrial production of Capecitabine or Cytarabine, intermediates or prodrugs thereof.

More preferably, the previously mentioned use is achieved by the production process and variants thereof, also previously detailed, of NAs as APIs, intermediates or prodrugs thereof.

**Comparative Example 1: Synthesis of 5-Fluoro-5'-deoxycytidine departing from unmodified 5-fluorocytosine and deoxyuridine**

A solution of 2.5 mM 5-fluorocytosine and 8.0 mM 5'-deoxyuridine in 30 mM aqueous phosphate buffer at pH 7 and 10% DMSO was heated at 60°C during 30 min. Then, PyNP (5.4 U/$\mu mol_{base}$) was added and the reaction was stirred at 60°C during 4 hours under the same conditions. Then, the crude reaction was filtered through a 10 KDa membrane, and a portion was diluted and analyzed by HPLC. The expected product 5-Fluoro-5'-deoxycytidine was not detected by UV-DAD (ultraviolet-diode array detection).

**Comparative Example 2: Synthesis of 5-Fluoro-5'-deoxycytidine departing from unmodified 5-fluorocytosine and chloro-5'-deoxyuridine**

A solution of 2.5 mM 5-fluorocytosine and 8.6 mM 5-chloro-5'-deoxyuridine in 30 mM aqueous phosphate buffer at pH 7 and 10% DMSO was heated at 60°C during 30 min. Then, PyNP (5.4 U/$\mu mol_{base}$) was added and the reaction was stirred at 60°C during 4 hours under the same conditions. Then, the crude reaction was filtered through a 10 KDa membrane, and a portion was diluted and analyzed by HPLC. The expected product 5-Fluoro-5'-deoxycytidine was not detected by UV-DAD.

**Comparative Example 3: Synthesis of Cytarabine departing from unmodified cytosine and arabinofuranosyluracil**

A solution of 3.0 mM cytosine and 10 mM 9-(b-D-arabinofuranosyl)uracil in 30 mM aqueous phosphate buffer at pH 7 and 10% DMSO was heated at 60°C during 30 min. Then, PyNP (4.4 U/$\mu mol_{base}$) was added and the reaction was stirred at 60°C during 4 hours under the same conditions. Then, the crude reaction was filtered through a 10 KDa membrane, and a portion was diluted and analyzed by HPLC. The expected product 1-(b-D-arabinofuranosyl)cytosine (Cytarabine) was not detected by UV-DAD, but uracil from cytosine decomposition through deamination was obtained.

**Comparative Example 4: Synthesis of Cytarabine departing from unmodified cytosine and arabinofuranosylcytosine**

A solution of 3.0 mM cytosine and 7.6 mM 9-(b-D-arabinofuranosyl)adenine in 30 mM aqueous phosphate

buffer at pH 7 and 10% DMSO was heated at 60°C during 30 min. Then, PyNP (4.4 U/$\mu$mol$_{base}$) was added and the reaction was stirred at 60°C during 4 hours under the same conditions. Then, the crude reaction was filtered through a 10 KDa membrane, and a portion was diluted and analyzed by HPLC. The expected product 1-(b-D-arabinofuranosyl)cytosine (Cytarabine) was not detected by UV-DAD, but hypoxanthine from adenine deamination and 9-(b-D-arabinofuranosyl)hypoxanthine from the susbtrate deamination were obtained.

**Comparative Example 5: Synthesis of Cytarabine departing from unmodified cytosine and arabinofuranosylhypoxanthine**

A solution of 3.0 mM cytosine and 7.5 mM 9-(b-D-arabinofuranosyl)hypoxanthine in 30 mM aqueous phosphate buffer at pH 7 and 10% DMSO was heated at 60°C during 30 min. Then, PyNP (4.4 U/$\mu$mol$_{base}$) was added and the reaction was stirred at 60°C during 4 hours under the same conditions. Then, the crude reaction was filtered through a 10 KDa membrane, and a portion was diluted and analyzed by HPLC. The expected product 1-(b-D-arabinofuranosyl)cytosine (Cytarabine) was not detected by UV-DAD.

Comparative Example 6: Synthesis of Cytarabine departing from unmodified **cytosine and arabinofuranosylguanine**

A solution of 3.0 mM cytosine and 8.6 mM 9-(b-D-arabinofuranosyl)guanine in 30 mM aqueous phosphate buffer at pH 7 and 10% DMSO was heated at 60°C during 30 min. Then, PyNP (4.4 U/$\mu$mol$_{base}$) was added and the reaction was stirred at 60°C during 4 hours under the same conditions. Then, the crude reaction was filtered through a 10 KDa membrane, and a portion was diluted and analyzed by HPLC. The expected product 1-(b-D-arabinofuranosyl)cytosine (Cytarabine) was not detected.

**Example 7: Synthesis of pentyl (5-fluoro-2-oxo-1,2-dihydropyrimidin-4-yl)carbamate**

Under inert atmosphere, pentyl chloroformate (5 g, 1.0 equiv) was added to a stirred solution of 5-fluorocytosine (1.0 equiv) in anhydrous pyridine, and the reaction was heated to 60 °C. After 2 hours, as no more conversion was observed, the reaction was quenched by cooling at room temperature. After that, the crude reaction was extracted using AcOEt and HCl 1 N, and the organic phase was dried over $Na_2SO_4$ and the solvent was evaporated. The white solid obtained was chromatographically purified using $SiO_2$ and AcOEt as mobile phase, yielding the desired product (88 %).

Example 8: Synthesis of ***N*-(2-oxo-1,2-dihydropyrimidin-4-yl)acetamide**

Under inert atmosphere, triethylamine (18,1 $\mu$L, 0,13 mmol) and acetyl chloride (9,2 $\mu$L, 0,13 mmol) were added to a stirred solution of cytosine (10,0 mg, 0,09 mmol) in anhydrous DMF at room temperature during 25 hours. After that, the solvent was evaporated, and the yellowish solid obtained was washed with water, filtered and dried *in vacuo,* rendering the expected product in 55% yield.

**Example 9: Synthesis of pentyl (2-oxo-1,2-dihydropyrimidin-4-yl)carbamate**

Under inert atmosphere, pentyl chloroformate (0.8 mmol, 1.0 equiv) was added dropwise to a stirred solution of cytosine (0.8 mmol, 1.0 equiv) in anhydrous pyridine, and the reaction was heated to 60 °C. After 2 hours, as no more conversion was observed, the reaction was quenched by cooling at room temperature. After that, the crude reaction was extracted using AcOEt and HCl 1 N, and the organic phase was dried over $Na_2SO_4$ and the solvent was evaporated. The white solid obtained was chromatographically purified using $SiO_2$ and AcOEt as mobile phase, yielding the desired product.

**Example 10: Synthesis of *N*$^4$-pentyloxycarbonyl- 5'-deoxy-5-fluorocytidine (Capecitabine) departing from uridine**

A solution of 2.7 mM pentyl (5-fluoro-2-oxo-1 ,2-dihydropyrimidin-4-yl)carbamate and 8.0 mM 5'-deoxyuridine in 30 mM aqueous phosphate buffer at pH 7 and 10% DMSO was heated at 60°C during 30 min. Then, PyNP (5.0 U/$\mu$mol$_{base}$) was added and the reaction was stirred at 60°C during 4 hours under the same conditions. Then, the crude reaction was filtered through a 10 KDa membrane, and a portion was diluted and analyzed by HPLC. The expected product *N*$^4$-pentyloxycarbonyl- 5'-deoxy-5-fluorocytidine (Capecitabine) was detected with a yield of 68%, in comparison to Reference Example 1, where the final product was not formed or detected.

**Example 11: Synthesis of $N^4$-pentyloxycarbonyl- 5'-deoxy-5-fluorocytidine (Capecitabine) departing from deoxyuridine.**

A solution of 2.5 mM pentyl (5-fluoro-2-oxo-1,2-dihydropyrimidin-4-yl)carbamate and 8.6 mM 5'-deoxyuridine in 30 mM aqueous phosphate buffer at pH 7 and 10% DMSO was heated at 60°C during 30 min. Then, PyNP (5.4 $U/\mu mol_{base}$) was added and the reaction was stirred at 60°C during 4 hours under the same conditions. Then, the crude reaction was filtered through a 10 KDa membrane, and a portion was diluted and analyzed by HPLC. The expected product $N^4$-pentyloxycarbonyl- 5'-deoxy-5-fluorocytidine (Capecitabine) was detected with a yield of 77%, in comparison to Reference Example 2, where the final product was not formed or detected.

**Example 12: Synthesis of 9-(b-D-arabinofuranosyl)uracil (Cytarabine) departing from arabinofuranosyluracil**

A solution of 2.5 mM pentyl (2-oxo-1,2-dihydropyrimidin-4-yl)carbamate and 8.6 mM 9-(b-D-arabinofuranosyl)uracil in 30 mM aqueous phosphate buffer at pH 7 and 10% DMSO was heated at 60°C during 30 min. Then, PyNP (5.4 $U/\mu mol_{base}$) was added and the reaction was stirred at 60°C during 4 hours under the same conditions. Then, the crude reaction was filtered through a 10 KDa membrane, and a portion was diluted and analyzed by HPLC. The expected product $N^4$-pentyloxycarbonylcytidine was detected.

$N^4$-position may be optionally deprotected to furnish 9-(b-D-arabinofuranosyl)uracil (Cytarabine).

**Example 13: Synthesis of 9-(b-D-arabinofuranosyl)uracil (Cytarabine) departing from arabinofuranosyladenine**

A solution of 2.5 mM pentyl (2-oxo-1,2-dihydropyrimidin-4-yl)carbamate and 8.6 mM 9-(b-D-arabinofuranosyl)adenine in 30 mM aqueous phosphate buffer at pH 7 and 10% DMSO was heated at 60°C during 30 min. Then, PyNP (5.4 $U/\mu mol_{base}$) was added and the reaction was stirred at 60°C during 4 hours under the same conditions. Then, the crude reaction was filtered through a 10 KDa membrane, and a portion was diluted and analyzed by HPLC. The expected product $N^4$-pentyloxycarbonylcytidine was detected.

$N^4$-position may be optionally deprotected to furnish 9-(b-D-arabinofuranosyl)uracil (Cytarabine).

**Claims**

1. Process for producing, by means of a method of either chemo-enzymatic or enzymatic synthesis, cytosine nucleoside analogues, intermediates or prodrugs thereof, of formula I

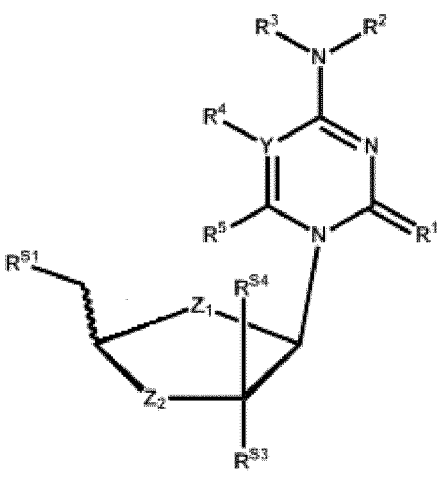

Formula I

wherein,

$Z_1$ being O, $CH_2$, S, NH;
$Z_2$ being, independently of $Z_1$: O, $C(R^{S2}R^{S5})$, $S(R^{S2}R^{S5})$, $S(R^{S2})$, $S(R^{S5})$, preferably, a group SO or $SO_2$; $N(R^{S2}R^{S5})$ $N(R^{S2})$, $N(R^{S5})$;
$R^{S1}$ being hydrogen, OH, ether or ester thereof selected from:

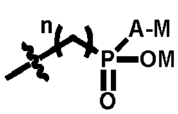 , 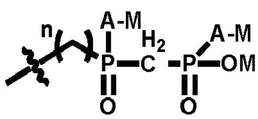 ,

being n is 0 or 1, A is oxygen or nitrogen, and each M is independently hydrogen, an optionally substituted alkyl chain, an optionally substituted alkenyl chain, an optionally substituted alkynyl chain, an optionally substituted aryl optionally linked to P by an optionally substituted alkyl, alkenyl or alkynyl chain, an optionally substituted heterocycle optionally linked to P by an optionally substituted alkyl, alkenyl or alkynyl chain, or a pharmaceutically acceptable counter-ion such as, but not restricted to, sodium, potassium, ammonium or alkylammonium;

$R^{S2}$ being hydrogen, OH or an ether or ester residue thereof, halogen, CN, $NH_2$, SH, $C\equiv CH$, $N_3$;

$R^{S3}$ being hydrogen, in case of NA derived from 2'-deoxyribonucleosides or arabinonucleosides or being selected from: OH, $NH_2$, halogen, $OCH_3$, when the NA is derived from ribonucleosides;

$R^{S4}$ being hydrogen, OH or an ether or ester residue thereof, $NH_2$, halogen, CN; providing $R^{S1}$ and $R^{S4}$ are different when both were ethers or esters of OH residues;

$R^{S5}$ being hydrogen, OH or an ether or ester residue thereof, $NH_2$ or halogen, in the case that $Z_2$ is different from oxygen;

$R^1$ being O, $CH_2$, S, NH;

$R^2$ being hydrogen, an optionally substituted $C_{4-40}$ alkyl chain, an optionally substituted alkenyl chain, an optionally substituted alkynyl chain, an optionally substituted aryl linked to N by an optionally substituted alkyl, alkenyl or alkynyl chain, an optionally substituted heterocycle linked to N by an optionally substituted alkyl, alkenyl or alkynyl chain, $COR^6$, $CONR^6R^7$, $CO_2R^6$, $C(S)OR^7$, CN, $SR^6$, $SO_2R^6$, $SO_2R^6$ $R^7$, CN, P(O)aryl, P(O)heterocycle, P(S)aryl, P(S)heterocycle, $P(O)O_2R^8$;

$R^3$ being hydrogen, an optionally substituted $C_{4-40}$ alkyl chain, an optionally substituted alkenyl chain, an optionally substituted alkynyl chain, , an optionally substituted aryl linked to N by an optionally substituted alkyl, alkenyl or alkynyl chain, an optionally substituted heterocycle linked to N by an optionally substituted alkyl, alkenyl or alkynyl chain, $COR^6$, $CONR^6R^7$, $CO_2R^6$, $C(S)OR^7$, CN, $SR^6$, $SO_2R^6$, $SO_2R^6$ $R^7$ CN, P(O)aryl, P(O)heterocycle, P(S)aryl, P(S)heterocycle, $P(O)O_2R^8$; being $R^3$ and $R^2$ independent one from each other; and providing that at least one $R^2$ or $R^3$ is different from hydrogen;

$R^4$ being hydrogen, OH, $NH_2$, SH, halogen; optionally substituted alkyl chain; optionally substituted alkenyl chain; optionally substituted alkynyl chain, trihaloalkyl, $OR^6$, $NR^6R^7$, CN, $COR^6$, $CONR^6R^7$, $CO_2R^6$, $C(S)OR^6$, $OCONR^6R^7$, $OCO_2R^6$, $OC(S)OR^6$, $NHCONR^6R^7$, $NHCO_2R^6$, $NHC(S)OR^6$, $SO_2NR^6R^7$, an optionally substituted aryl linked to Y by an optionally substituted alkyl, alkenyl or alkynyl chain, an optionally substituted heterocycle linked to Y by an optionally substituted alkyl, alkenyl or alkynyl chain, and any optionally substituted heterocycle or optionally substituted aryl of, independently, $R^2$, $R^3$, $R^4$ or $R^5$, selected from:

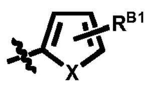 , 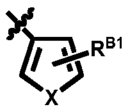 , 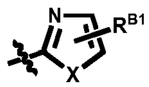 , 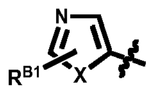 ,

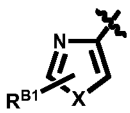 , 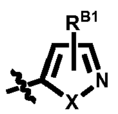 , 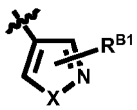 , 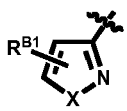 ,

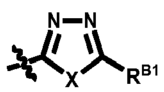 , 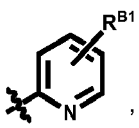 , 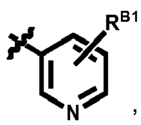 , 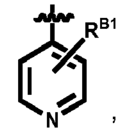 ,

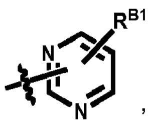 , 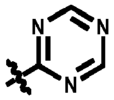 , 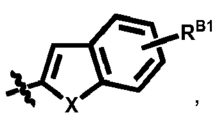 , 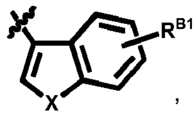 ,

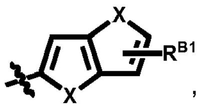 , 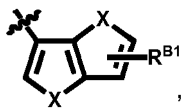 , 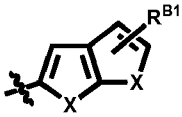 , 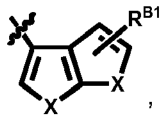 ,

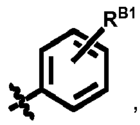 ,

providing Y is a carbon or sulphur atom and, alternatively, $R^4$ being absent, providing Y is a nitrogen atom; wherein

X being O, S, N-$R^{B2}$, Se; $R^{B1}$ being H, OH, $NH_2$, SH, straight or branched $C_{1-10}$ alkyl, F, Cl, Br, I, X-$R^{B2}$, -C≡C-$R^{B2}$, $CO_2R^{B2}$; $R^{B2}$ being H, OH, $NH_2$, straight or branched $C_1$-$_5$ alkyl, phenyl;

$R^5$ being hydrogen, OH, $NH_2$, SH, halogen, an optionally substituted alkyl chain, an optionally substituted alkenyl chain, an optionally substituted alkynyl chain, trihaloalkyl, OR , $NR^6R^7$, CN, $COR^6$, $CONR^6R^7$, $CO_2R^6$, C(S)$OR^6$, $OCONR^6R^7$, $OCO_2R^6$, OC(S)$OR^6$, $NHCONR^6R^7$, $NHCO_2R^6$, NHC(S)$OR^6$, $SO_2NR^6R^7$;$CH_2$-heterocyclic ring, CN;

and any optionally substituted heterocycle or optionally substituted aryl of, independently, $R^2$, $R^3$, $R^4$ or $R^5$, selected from:

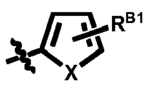 , 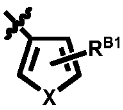 , 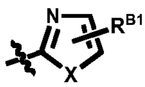 , 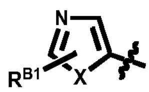 ,

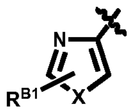 , 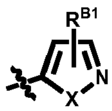 , 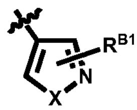 , 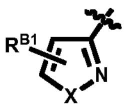 ,

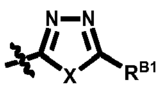 , 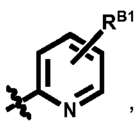 , 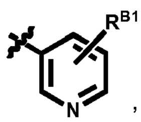 , 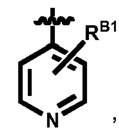 ,

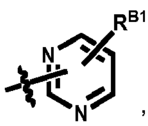 , 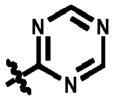 , 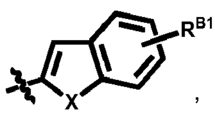 , 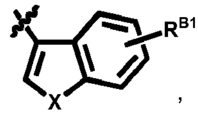 ,

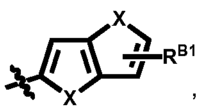 , 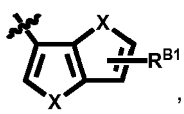 , 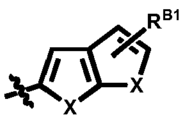 , 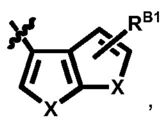 ,

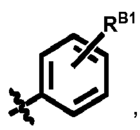 ,

wherein
X being O, S, N-$R^{B2}$, Se; $R^{B1}$ being H, OH, $NH_2$, SH, straight or branched $C_{1-10}$ alkyl, F, Cl, Br, I, X-$R^{B2}$, -C≡C-$R^{B2}$, $CO_2R^{B2}$; $R^{B2}$ being H, OH, $NH_2$, straight or branched $C_{1-5}$ alkyl, phenyl;
$R^6$ and $R^7$ are independently of each other hydrogen, optionally substituted alkyl chain, optionally substituted alkenyl chain, optionally substituted alkynyl chain, heterocyclic or optionally substituted aryl;
$R^8$ being hydrogen, an optionally substituted alkyl chain, an optionally substituted alkenyl chain, an optionally substituted alkynyl chain, an optionally substituted aryl an optionally substituted heterocycle;
Y being C, N, S;

wherein the process for producing the cytosinic nucleoside analogue comprises the following steps:

(i) chemically reacting a precursor of the cytosinic nucleobase of formula II, wherein Y, $R^1$, $R^4$, $R^5$, $R^6$ and $R^7$, are defined as above, with suitable reagents for modifying its amino group at $N^4$ position in order to incorporate the proper substitution described as substituent $R^2$ and $R^3$, said modified cytosinic nucleobase of formula II formed thereof, being optionally purified by conventional purification methods; or alternatively, the process departs directly from the starting products represented by a cytosinic nucleobase of formula II as such.

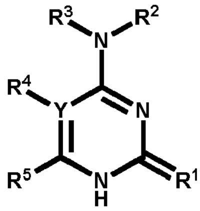

Formula II

(ii) biocatalytically reacting the above mentioned modified cytosinic nucleobase of formula II, with a suitable nucleoside analog substrate of formula III,

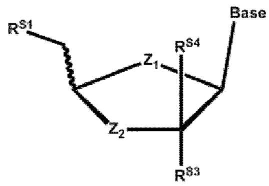

Formula III

wherein $Z_1$, $Z_2$, $R^{S1}$, $R^{S2}$, $R^{S3}$, $R^{S4}$, $R^{S5}$ are defined as above and the Base is selected from: uracil, adenine, cytosine, guanine, thymine, hypoxanthine, xanthine, thiouracil, thioguanine, 9-*H*-purine-2-amine, 7-methylguanine, 5-fluorouracil, 5-bromouracil, 5-chlorouracil, 5,6-dihydrouracil, 5-methylcytosine and 5-hydroxymethylcytosine, pteridone, and any substituted derivative thereof;
wherein the aforesaid reaction carried out in step ii), comprises the addition, in a suitable reaction aqueous medium and under suitable reaction conditions, of a nucleoside phosphorylase enzyme, either a Pyrimidine Nucleoside Phosphorylase enzyme, a Purine Nucleoside Phosphorylase enzyme or combinations thereof, to a mixture of starting materials comprising a cytosine nucleobase of formula II and a nucleoside analogue of formula III,
(iii) optionally, deprotecting the amino group at $N^4$ position in cytosinic nucleoside analogue in order to recover the free primary amino $N^4$ at the cytosinic nucleoside analogue of formula I which was further purified by conventional purification methods.

**2.** A process according to claim 1, wherein the cytosinic nucleobase of formula II to be transferred by the Pyrimidine Nucleoside Phosphorylase enzyme is selected from:

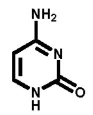

4-aminopyrimidin-2(1*H*)-one (cytosine)

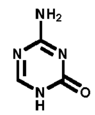

4-amino-1,3,5-triazin-2(1*H*)-one (5-azacytosine)

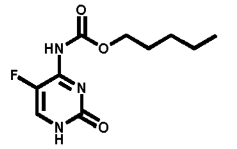

pentyl (5-fluoro-2-oxo-1,2-dihydropyrimidin -4-yl)carbamate

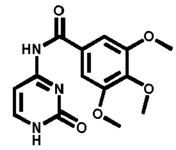

3,4,5-trimethoxy-*N*-(2-oxo-1,2-dihydro-pyrimidin-4-yl)benzamide

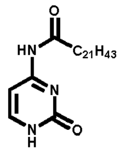

*N*-(2-oxo-1,2-dihydropyrimidin-4-yl) docosanamide

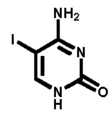

4-amino-5-iodopyrimidin-2(1*H*)-one (5-iodocytosine)

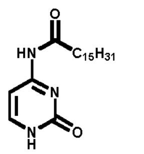

*N*-(2-oxo-1,2-dihydropyrimidin-4-yl) palmitamide

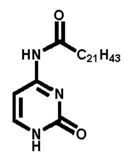

*N*-(2-oxo-1,2-dihydropyrimidin-4-yl) heneicosamide

3. The process according to any of the claims 1 or 2, wherein the nucleoside analogs, intermediates or prodrugs thereof produced are selected from: Capecitabine, Decitabine, 5-Azacytidine, Cytarabine, Enocitabine, Gemcitabine, Zalcitabine, Ibacitabine, Sapacitabine, 2'-C-cyano-2'-deoxy-1-β-D-arabino-pentofuranosylcytosine, Galocitabine, Valopricitabine, 2'-Deoxy-4'-thiocytidine, Thiarabine, 2'-Deoxy-4'-thio-5-azacytidine and Apricitarabine.

4. The process according to any of the claims 1 to 3 wherein the source of native and mutants or variants thereof, or of recombinant nucleoside phosphorylase enzyme, are mesophilic, thermophilic or hyperthermophilic organisms.

5. The process according to any of the claims 1 to 4 wherein the source of native and mutants or variants thereof, or of recombinant nucleoside phosphorylase enzyme is selected from *Archaea* or bacteria.

6. The process according to claim 5 wherein the nucleoside phosphorylase enzyme is isolated from an *Archaea* selected from: *Sulfolobus solfataricus* or *Aeropyrum pernix.*

7. The process according to claim 1 wherein the nucleoside phosphorylase enzyme, or a functional part thereof, is encoded by a nucleotide sequence selected from: SEQ ID NO. 1, 2, 5, 7, 9 or 11; or

   a) a nucleotide sequence which is the complement of SEQ ID. NO:1, 2, 5, 7 , 9 or 11 ; or
   b) a nucleotide sequence which is degenerate with SEQ ID. NO:1, 2, 5, 7 , 9 or 11 ; or
   c) a nucleotide sequence hybridizing under conditions of high stringency to SEQ ID. NO:1, 2, 5, 7 , 9 or 11; to the complement of SEQ ID. NO:1, 2, 5, 7 , 9 or 11; or to a hybridization probe derived from SEQ ID. NO:1, 2, 5, 7 , 9 or 11; or their complement thereof ; or
   d) a nucleotide sequence having at least 80% sequence identity with SEQ ID. NO:1,2,5,7,9 or 11; or
   e) a nucleotide sequence having at least 59% sequence identity with SEQ ID. NO:1,2,5,7,9 or 11; or
   f) a nucleotide sequence encoding for an amino acid sequence selected from: SEQ ID. NO: 3, 4, 6, 8, 10 or 12.

8. The process according to claim 3 wherein the nucleoside analogue produced is, Capecitabine, the ribonucleoside used as starting material is selected from: 5'-deoxyuridine, 5'-deoxy-5-methyluridine or 5'-deoxy-5-chlorouridine; and the nucleobase also used as starting material to be transferred by the Pyrimidine Nucleoside Phosphorilase enzyme, is pentyl (5-fluoro-2-oxo-1,2-dihydropyrimidin-4-yl)carbamate.

9. The process according to claim 3 wherein the nucleoside analogue produced is, Cytarabine, the ribonucleoside used as starting material is 9-(b-D-arabinofuranosyl)uracil and the nucleobase also used as starting material to be transferred by the Pyrimidine Nucleoside Phosphorilase enzyme, is N-(2-oxo-1,2-dihydropyrimidin-4-yl) pentanamide.

10. Use of a mesophilic, thermophilic or hyperthermophilic nucleoside phosphorylase, either native and mutants or variants thereof, or of recombinant enzyme, or a functional part thereof; or of a recombinant expression vector comprising a sequence encoding a natural or recombinant, mesophilic, thermophilic or hyperthermophilic, nucleoside phosphorylase, or a functional part thereof, operably linked to one or more control sequences that direct the expression or overexpression of said nucleoside phosphorylase in a suitable host; or of a microorganism or a host cell containing the sames, in the production of cytosinic nucleoside analogues, intermediates or prodrugs thereof, useful

as anti-cancer or anti-viral medicaments.

11. Use according to claim 10 wherein the mesophilic, thermophilic or hyperthermophilic nucleoside phosphorylase enzyme, or a functional part thereof, is encoded by a nucleotide sequence selected from: SEQ ID NO. 1, 2, 5, 7, 9 or 11; or

a) a nucleotide sequence which is the complement of SEQ ID. NO:1, 2, 5, 7 , 9 or 11 ; or
b) a nucleotide sequence which is degenerate with SEQ ID. NO:1, 2, 5, 7 , 9 or 11 ;or
c) a nucleotide sequence hybridizing under conditions of high stringency to SEQ ID. NO:1, 2, 5, 7 , 9 or 11; to the complement of SEQ ID. NO:1, 2, 5, 7 , 9 or 11; or to a hybridization probe derived from SEQ ID. NO:1, 2, 5, 7 , 9 or 11; or their complement thereof ; or
d) a nucleotide sequence having at least 80% sequence identity with SEQ ID. NO:1,2,5,7,9 or 11; or
e) a nucleotide sequence having at least 59% sequence identity with SEQ ID. NO:1,2,5,7,9 or 11; or
f) a nucleotide sequence encoding for an amino acid sequence selected from: SEQ ID. NO: 3, 4, 6, 8, 10 or 12.

12. Use according to any of the claims 10 or 11, wherein the cytosinic nucleoside analogues, intermediates or prodrugs thereof produced are selected from: Capecitabine, Decitabine, 5-Azacytidine, Cytarabine, Enocitabine, Gemcitabine, Zalcitabine, Ibacitabine, Sapacitabine, 2'-C-cyano-2'-deoxy-1-β-D-arabino-pentofuranosylcytosine, Galocitabine, Valopricitabine, 2'-Deoxy-4'-thiocytidine, Thiarabine, 2'-Deoxy-4'-thio-5-azacytidine and Apricitarabine.

13. Use according to claim 12, wherein the cytosinic nucleoside analogue, intermediates or prodrugs thereof produced is Capecitabine.

14. Use according to claim 12, wherein the cytosinic nucleoside analogue, intermediates or prodrugs thereof produced is Cytarabine.

Europäisches Patentamt
European Patent Office
Office européen des brevets

# EUROPEAN SEARCH REPORT

Application Number
EP 13 19 7287

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | MIKHAILOPULO ET AL: "Biologically important nucleosides: modern trends in biotechnology and application", MENDELEEV COMMUNICATIONS, vol. 21, 2011, pages 57-68, XP028189733, * See the reaction schemes * | 1-14 | INV. C12P19/30 |
| A | DING ET AL: "Induction of recombinant uridine phosphorylase and its application in biosynthesis of pyrimidine nucleosides", BIOTECHNOLOGY AND BIOENGINEERING / CHINESE JOURNAL OF CHEMICAL ENGINEERING, vol. 19, 2011, pages 122-127, XP002725012, * See page 125 (Figure 5) * | 1-14 | |
| A,D | DING ET AL: "Enzymatic synthesis of nucleosides by nucleoside phosphorylase co-expressed in Escherichia coli", JOURNAL OF ZHEJIANG UNIVERSITY - SCIENCE B, vol. 11, 2011, pages 880-888, XP002725013, * See page 881 (Figure 1) and page 886 (Table 4) * | 1-14 | TECHNICAL FIELDS SEARCHED (IPC): C12P |
| A,D | FRESCO-TABOADA ET AL: "New insights on nucleoside 2'-deoxyribosyltransferases: a versatile biocatalyst for one-pot one-step synthesis of nucleoside analogs", APPLIED MICROBIOLOGY AND BIOTECHNOLOGY, vol. 97, 26 March 2013 (2013-03-26), pages 3773-3785, XP002725030, * See page 3773 (Abstract); early online publication * | 1-14 | |

-/--

The present search report has been drawn up for all claims

| Place of search | Date of completion of the search | Examiner |
|---|---|---|
| Berlin | 27 May 2014 | Korsner, Sven-Erik |

2

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 13 19 7287

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | WO 2011/076894 A1 (INSTITUT UNIV. DE CIÈNCIA I TECNOLOGIA, S.A.) 30 June 2011 (2011-06-30)<br>* See Abstract and Figure 7 (= SEQ 1 of the present Application) *<br>----- | 1-14 | |

TECHNICAL FIELDS SEARCHED (IPC)

The present search report has been drawn up for all claims

| Place of search | Date of completion of the search | Examiner |
|---|---|---|
| Berlin | 27 May 2014 | Korsner, Sven-Erik |

2

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 13 19 7287

This annex lists the patent family members relating to the patent documents cited in the above-mentioned European search report.
The members are as contained in the European Patent Office EDP file on
The European Patent Office is in no way liable for these particulars which are merely given for the purpose of information.

27-05-2014

| Patent document cited in search report | Publication date | Patent family member(s) | Publication date |
|---|---|---|---|
| WO 2011076894 A1 | 30-06-2011 | CA 2785229 A1 | 30-06-2011 |
| | | CN 102770532 A | 07-11-2012 |
| | | EP 2338985 A1 | 29-06-2011 |
| | | EP 2516637 A1 | 31-10-2012 |
| | | JP 2013514797 A | 02-05-2013 |
| | | KR 20130027452 A | 15-03-2013 |
| | | RU 2012125367 A | 27-01-2014 |
| | | US 2012264175 A1 | 18-10-2012 |
| | | US 2013337547 A1 | 19-12-2013 |
| | | WO 2011076894 A1 | 30-06-2011 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 1254959 A, Araki
- US 7629457 B
- US 20050074857 A, Araki
- JP 2004344029 B
- JP 2004024086 B
- EP 0602454 A, Arasaki
- EP 0602478 A, Kamiya
- WO 2011104540 A
- WO 2010061402 A, Kanan
- NL 6511420, Merck
- WO 2011076894 A


### Non-patent literature cited in the description


- **BORYSKI J.** Reactions of transglycosylation in the nucleoside chemistry. *Curr Org Chem,* 2008, vol. 12, 309-325
- Enzymatic synthesis of modified nucleosides. **CONDEZO, L. A. et al.** Biocatalysis in the pharmaceutical and biotechnology industries. CRC Press, 2007, 401-423
- Enzyme-catalyzed synthesis of nonnatural or modified nucleosides. **SINISTERRA, J.V. et al.** Encyclopedia of Industrial Biotechnology: Bioprocess, Bioseparation, and Cell Technology. John Wiley & sons, 2010, 1-25
- **MIKHAILOPULO, I. A et al.** New Trends in Nucleoside Biotechnology. *Acta Naturae,* 2010, vol. 2 (5), 36-58
- **FRESCO-TABOADA et al.** New insights on NDTs: a versatile Biocatalyst for one-pot one-step synthesis of nucleoside analogs. *Appl. Microbiol. Biotechnol,* 2013, vol. 97, 3773-3785
- **DING Q.** Enzymatic synthesis of nucleosides by mucleoside phosphorylase co-expressed in Escherichia coli. *J. Zheijiang Univ-Sci B,* 2010, vol. 11 (11), 880-888
- **SZEKER, K.** Nucleoside phosphorylases from thermophiles. *Ph.D. Thesis,* 2012
- *Salt lake Institute of Biological Studies,* 1969
- The Pharmacological Basis of Therapeutics. **GOODMAN ; GILMAN.** Biotransformation of Drugs. McGraw-Hill, 1992, 13-15
- **SAMBROOK et al.** Molecular Cloning, A Laboratory Manual. Cold Spring Harbor Laboratory Press, 1989